(19)

**Europäisches Patentamt**

**European Patent Office**

**Office européen des brevets**

(11) **EP 4 446 406 A1**

(12) **EUROPEAN PATENT APPLICATION**

(43) Date of publication:
**16.10.2024   Bulletin 2024/42**

(21) Application number: **24169761.4**

(22) Date of filing: **11.04.2024**

(51) International Patent Classification (IPC):
*C12N 1/20* (2006.01)      *C12P 19/04* (2006.01)
*C08B 16/00* (2006.01)      *C08J 11/10* (2006.01)

(52) Cooperative Patent Classification (CPC):
**C12N 1/20; C08J 11/105; C08J 11/16; C08J 11/28;
C12P 19/04;** C08J 2301/00; C12P 2201/00;
C12P 2203/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:   **12.04.2023   EP 23167596**

(71) Applicant: **Sanko Tekstil Isletmeleri San. Ve Tic.
A.S.
16400 Inegol - Bursa (TR)**

(72) Inventors:
• **Akbas, Halil Ibrahim
16400 Inegol - BURSA (TR)**

• **Özseyhan, Mehmet Erkan
16400 Inegol - BURSA (TR)**
• **Öktem, Gözde
16400 Inegol - BURSA (TR)**
• **Kansu, Melih
16400 Inegol - BURSA (TR)**
• **Kardes, Zeynep
16400 Inegol - BURSA (TR)**
• **Kazanç, Semih
16400 Inegol - BURSA (TR)**

(74) Representative: **Gislon, Gabriele
Marietti, Gislon e Trupiano S.r.l.
Via Larga, 16
20122 Milano (IT)**

(54) **PROCESS FOR THE PRODUCTION OF MICROBIAL CELLULOSE USING WASTE TEXTILES**

(57)      The present invention refer to a process for the production of microbial cellulose using a waste textile material, for example 100% cotton or blended cotton/polyester/elastane waste textile, said waste textile material comprising cellulose, comprising the following steps: providing an amount of said waste textile material; pre-treating said waste textile material with an aqueous solution comprising NaOH and urea, to obtain a pre-treated fabric, wherein said step of treating said waste textile with an aqueous solution comprising NaOH and urea is carried out at a temperature ranging from -25°C to +30°C; treating said pre-treated fabric with cellulase enzymes, to convert said cellulose into glucose, whereby a mixture including glucose is obtained; preparing a culture medium comprising said mixture including glucose; inoculating said culture medium with microbial cellulose producing microorganisms; and incubating said microbial cellulose producing microorganisms to obtain microbial cellulose.

Fig. 1

EP 4 446 406 A1

**Description**

Field of the invention

The present invention relates to a process for the production of microbial cellulose using waste textile materials. In greater detail, the present invention relates to a process for the production of microbial cellulose starting from waste textile materials including cellulose fibers and, optionally, also synthetic fibers.

State of the art

The microbial cellulose is one of the most known and studied bacterial biopolymers. The microbial cellulose is an organic compound with the formula $(C_6H_{10}O_5)_n$, the same of plant cellulose, that is produced from certain types of microorganisms, such as bacteria, as extracellular polymer. Although the microbial cellulose, e.g., the bacterial cellulose, has the same molecular formula of the plant cellulose, it differs from the latter one in macromolecular properties. In fact, microbial cellulose is generally more chemically pure with respect to plant cellulose (i.e., it contains no hemicellulose or lignin), and it has a higher water holding capacity, a greater tensile strength, a higher degree of polymerization and a higher crystallinity. Due to these peculiar properties, the microbial cellulose has been applied in several technical fields, such as food industry, medical field (e.g., as wound dressing and in blood vessel regeneration) and paper restoration. The application of microbial cellulose (e.g., bacterial cellulose) in the field of textiles is also known.

Recycling used textiles, also known as "waste textiles", has become a primary target in the textile industry; new and efficient processes for recycling are being actively researched. One of the goals is to recycle the materials included in the waste textiles, e.g., the fibers included in the waste textile, for example, by converting the fibers of waste textiles into raw materials suitable to be used in different processes, such as processes for the production of microbial cellulose.

Processes for obtaining bacterial cellulose starting from waste textile materials are known in the art. Within these processes it is known to pre-treat a fabric including yarns containing cotton fibers with organic salts such as N-methylmorpholine-N-oxide (NMMO) or 1-alkyl-3-methylimidazolium ionic liquids, before the enzymatic hydrolysis.

For example, CN102080114 discloses a method for preparing bacterial cellulose from waste cotton fabrics. The method according to CN102080114 includes a step of pre-treating the cotton fabrics in an ionic liquid at the temperature of 90°C-130°C for a time from 15 minutes to 10 hours. Cellulase enzymes are, then, added for enzyme hydrolysis to obtain an enzymolysis liquid. The enzymolysis liquid is used to prepare a culture medium for bacterial cellulose producing bacteria.

CN111269953 discloses a method for preparing bacterial cellulose from waste polyester fabrics, which comprises a step of adding a barium hydroxide solution, and treating the fabric at a temperature of 120°C to 126°C for 90-100 minutes. After the hydrolysis is finished, sulfuric acid is added until no precipitate is generated, the pH value of the solution is adjusted to be neutral, and centrifugal separation is carried out to obtain a supernatant, namely polyester hydrolysate. The polyester hydrolysate is used as a source of carbon to prepare a culture medium for bacteria producing bacterial cellulose.

The currently available processes for obtaining microbial cellulose starting from waste textile materials have several drawbacks.

For example, currently available processes generally result in a reduced efficiency of the enzymatic conversion into glucose; the applicant has realized that this reduced efficiency is due to the presence of dissolved cellulose in the liquid phase obtained after pre-treatment and/or to an at least partial degradation of the cellulose fibers before the conversion of cellulose into glucose. For example, it is known from CHIA-HUNG KUO ET AL, "Enzymatic saccharification of dissolution pretreated waste cellulosic fabrics for bacterial cellulose production by Gluconacetobacter xylinus", JOURNAL OF CHEMICAL TECHNOLOGY AND BIOTECHNOLOGY, vol. 85, no. 10, pages 1346-1352, that the treatment of fabrics with ionic liquids results in the dissolution of cellulose. When cellulose-containing textiles are treated with ionic liquids (such as NMMO or 1-butyl-3-methylimidazolium chloride ([BMIM]Cl)) or strong acids (e.g., phosphoric acid and solution thereof), the vast majority of cellulose is dissolved, thus requiring a regeneration step to obtain cellulose in a solid form before the treatment with cellulase enzymes. Therefore a solution of cellulose is not a suitable starting material for enzymatic conversion of cellulose to glucose.

In addition to the above problems, currently available processes carried out on waste textiles including both cellulosic fibers (e.g., cotton, lyocell and viscose fibers) and non-cellulosic (e.g., synthetic fibers, such as polyester fibers) result in an at least partial degradation of the non-cellulosic material, e.g., the polyester fibers. Degradation of the polyester fibers by hydrolysis of the polymer may be detrimental to the possibility of re-using the polyester fibers by melting them to extrude new polyester fibers having the required mechanical characteristics.

On the other side, it is necessary to provide ways of increasing the speed of enzymatic conversion of the cellulose fibers of the fabric into glucose and to reduce the overall cost of the circular recycling process that starts from cellulosic fibers and ends with (recycled) cellulosic fibers.

Furthermore, currently available processes require a great amount of energy, for example, in view of the conditions of said processes. Also, reagents used in the currently available processes (e.g., ionic liquids) require expensive chemical processes to be recovered and possibly reused.

Summary of the invention

An aim of the present invention is to solve the above problem and to provide a process for the production of microbial cellulose using waste textiles in an effective way. In particular it is an aim of the invention to provide a process for saccharification of cellulose that is efficient and easy to apply on industrial scale.

Another aim of the present invention is to provide a process for the production of microbial cellulose using waste textiles that is suitable to be carried out on waste textiles including both cellulosic fibers and non-cellulosic, synthetic, fibers, in particular polyester fibers; the process should provide a final product consisting of or including synthetic fibers, e.g. polyester fibers, substantially free from cotton and cellulosic fibers. The process should also reduce or help preventing depolymerization of the polyester or other synthetic fibers present in the textile that undergoes the invention treatment.

Also aim of the present invention is to provide a process for the production of microbial cellulose using waste textiles that allows to reduce energy consumption.

These and other aims are achieved by means of the present invention, which relates to a process according to claim 1, as well as to a process of enzymatic saccharification of cellulosic fabrics according to claim 2 and to a process of recycling blend textiles according to claim 19. Preferred embodiments of the invention are according to the dependent claims.

Brief description of the drawings

Figure 1 schematically illustrates an embodiment of the process of the invention.
Figure 2 shows SEM images of an exemplary waste textile material before and after a pre-treatment according to the process of the invention.
Figure 3A and Figure 3B show the microscope images of an exemplary bacterial cellulose obtained according to the process of the invention, in two magnifications.
Figure 4A and Figure 4B show SEM images of a 100% polyester fabric a pre-treatment carried out using an aqueous solution comprising NaOH and urea at room temperature according to the invention (Figure 4A) and after (Figure 4B) a pre-treatment carried out using an aqueous solution comprising NaOH and urea at a temperature of 50°C.

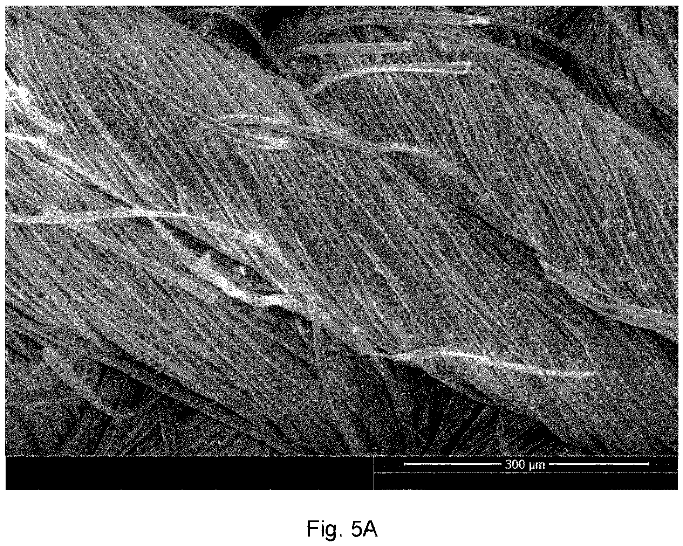

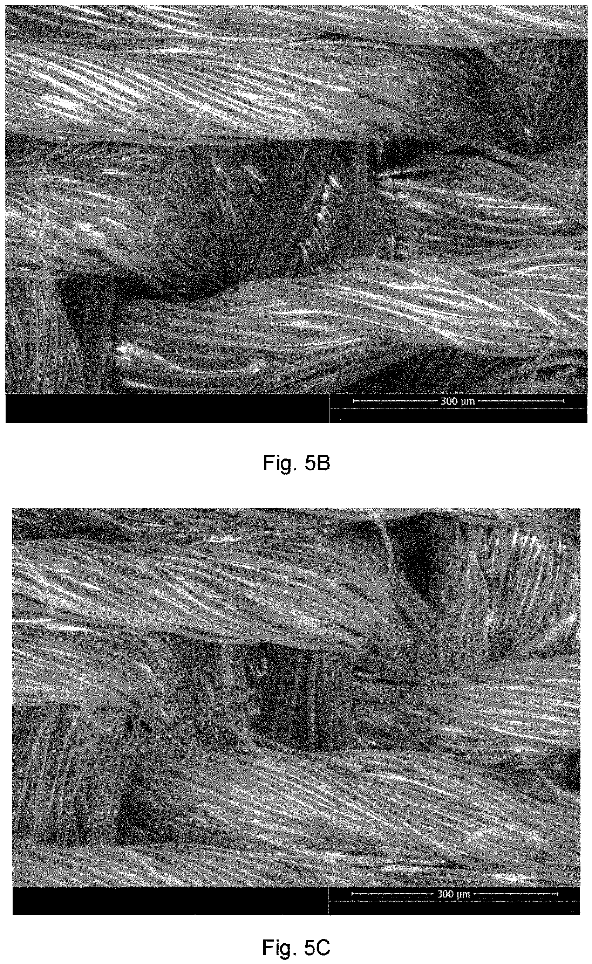

Figure 5A, Figure 5B and Figure 5C show SEM images of an exemplary waste textile before (Figure 5A) and after (Figure 5B and Figure 5C) a pre-treatment and an enzymatic treatment according to the process of the invention. Particularly, Figure 5B shows an exemplary waste textile material after a pre-treatment carried out at 0°C and a treatment with cellulase, according to embodiments of the process of the invention; Figure 5C shows an exemplary waste textile material after a pre-treatment carried out at room temperature and a treatment with cellulase, according to embodiments of the process of the invention.

Detailed description of the invention

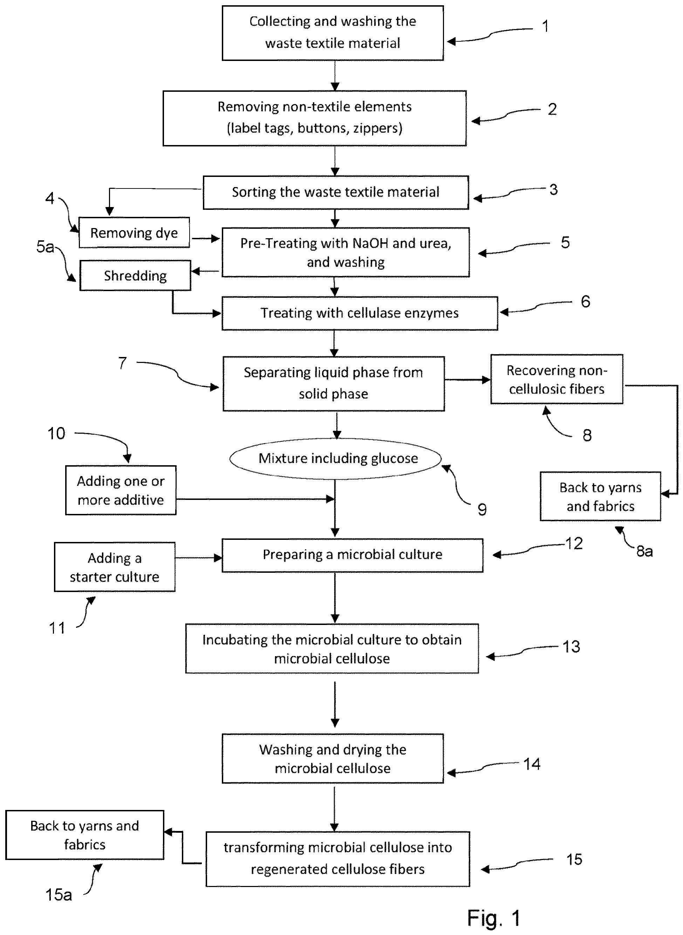

As shown in figure 1, according to an aspect, the present invention relates to a process for the production of microbial cellulose from a waste textile material, said waste textile material comprising cellulose in the form of cellulosic fibers, comprising the following steps:

a) providing a waste textile material;
b) pre-treating said waste textile material with an aqueous solution comprising a base, preferably NaOH, and an amide, preferably urea, to obtain a pre-treated textile material, wherein said step of pre-treating said waste textile material is carried out at a temperature within the range from -25°C to +30°C;
c) treating said pre-treated textile material with cellulase, to convert said cellulose into glucose, whereby a mixture including a solid phase and a liquid phase is obtained;
d) separating said liquid phase from said solid phase, to obtain a liquid phase mixture including glucose;
e) preparing a microbial culture using said liquid phase mixture including glucose, wherein said microbial culture comprises microbial cellulose-producing microorganisms;
f) incubating said microbial culture to obtain microbial cellulose.

Advantageously, the present invention allows to obtain microbial cellulose, starting from waste textile materials in an effective way. Also, the present invention allows to obtain microbial cellulose, starting from waste textile materials in an energy saving and, thus, cost saving way.

In a possible embodiment, the microbial cellulose obtained with the invention process is used as a starting cellulose material in a viscose-like process that transforms the microbial cellulose into regenerated cellulosic fibers.

The presence of NaOH (or another base) and urea (or another amide, e.g. thiourea) apart for cost-effectiveness with an affordable cellulose pretreatment solution, provides advantages such as improved cellulose properties that enhance the following steps of the process of production of microbial cellulose. In greater detail it was found that the use of the above cited reagents at the specified temperatures, and in particular at room temperature, facilitates enzymatic degradation and increases the yield of glucose available after enzymatic saccharification. It was also surprisingly found that the conversion yield of cellulose to glucose is higher when the textile, namely the fabric, to be treated is shredded (or in general it is subjected to a process of reduction of its size) after it has undergone the step of pre-treatment with NaOH/urea; preferably, the textile is dried before it is shredded.

It is believed that a first reason is that the amount of cellulose that is dissolved during the pre-treatment with NaOH and urea is little enough that it does not jeopardize or significantly reduce the enzymatic activity of the cellulase in the following step. Additionally, since only a very small amount of cellulose is dissolved during the pre-treatment with NaOH and urea of the fabric, especially when the treated fabrics have large areas, before shredding, a regenerating step to regenerate, i.e. to precipitate cellulose as a solid after its dissolution by adding water to the cellulose solution, is not required. Further reasons are believed to be that the addition of urea can trap free water and prevent interaction between cellulose chains through hydrogen bonds and that the treatment provides a reduction in the crystallinity of cellulose and a negligible change in polymerization of degree of cellulose.

The present invention, thus, further relates to a process of enzymatic saccharification of a waste textile material, said waste textile material comprising cellulosic fibers including cellulose, said process comprising the following steps:

a) providing a waste textile material;
b) pre-treating said waste textile material with an aqueous solution comprising NaOH and urea, to obtain a pre-treated textile material, wherein said step of pre-treating said waste textile material with an aqueous solution comprising NaOH and urea is carried out at a temperature in the range from -25°C to +30°C;
c) treating said pre-treated textile material with cellulase, to convert said cellulose into glucose, whereby a mixture including a solid phase and a liquid phase is obtained,

wherein said pre-treated textile as obtained in step b) is shred into a plurality of pieces having dimension suitable for enzymatic saccharification before step c).

As a matter of fact, the pre-treatment used in the process of the invention results in a reduced amount of enzyme being required for the subsequent enzymatic conversion of the cellulosic fibers into glucose, i.e. in an amount of enzyme low enough to make it economically feasible to renounce recovering the enzymes after the enzymatic reaction. This effect results in the possibility of using the glucose containing solution as a basis for the production of microbial cellulose without removing the cellulase enzymes: the cellulase enzymes are in fact inactivated before cellulose producing microorganisms are added to the glucose + enzyme solution and provide a source of Nitrogen and Carbon for the biocellulose production.

In embodiments, the base is selected from sodium hydroxide (NaOH), potassium hydroxide (KOH), calcium hydroxide $Ca(OH)_2$, lithium hydroxide (LiOH), and sodium carbonate $(Na_2CO_3)$.

In embodiments, the amide is selected from urea, and thiourea. In a preferred embodiment of the pre-treatment solution the ratio by weight of base/amide, namely NaOH/urea is in the range of 1.0/1.0 to 1.0/2.0, preferably 1.0/1.5 to 1.0/2.0, more preferably 1.0/1.5 to 1.0/1.8; a preferred ratio is about 1.0/1.7.

Energy savings are obtained in view of the fact that the pre-treating step is carried out using an aqueous solution comprising a base, preferably NaOH, and an amide, preferably urea, at a temperature ranging from -25°C to +30°C. A suitable temperature is in the range of -25°C to +25°C, preferably -10°C to +25°C, more preferably at +10°C to +25°C, most preferably at room temperature, i.e. at a temperature of 20°C ± 5°C. In fact, the use of an aqueous solution comprising NaOH and urea at the above temperature, preferably in the range from +15°C to +25°C allows to obtain a pre-treated fabric in which the yarns in a fabric are swollen (the fibers are not dissolved) and space is generated between the cellulosic fibers that thus become better available to the cellulase enzymes. At the same time, the level of degradation and the level of dissolution of the cellulose fibers are kept to a minimum. A further advantage of the invention process is that in a blend textile, e.g. a blend fabric, the process results in minimizing the possible damage to the polyester parts of the blend textile, i.e. degradation of the polyester filaments or fibers is kept to a minimum in order to recover polyester material for being recycled.

In the present process, NaOH and urea solution modifies the crystalline cellulose structure by converting cellulose I structure to cellulose II, as in mercerization, hydrolyzing the amorphous regions of cellulose. Advantageously, it has been observed that cellulase enzymes are more effective in converting cellulose to glucose when cellulose has structure II, with respect to cellulose having structure I. Native cotton cellulose is found to be mostly in Cellulose I structure. After NaOH/urea pre-treatment, cellulose results to be mostly in Cellulose II structure. As above mentioned, cellulose structure I is more difficult to be converted to glucose by cellulase enzymes when compared with Cellulose II. Indeed, Cellulose structure I is more resistant to enzymatic hydrolysis. Cellulose I has a highly ordered structure, which makes it more difficult for cellulase enzymes to access and hydrolyze the cellulose chains. On the other hand, Cellulose II has a more disordered structure, which makes it more accessible to cellulase enzymes and easier to hydrolyze. Without being bound to a specific scientific explanation, it has been observed that pre-treating using an aqueous solution of NaOH (or another base) and urea (or another amide), can be used to convert Cellulose I to Cellulose II, thereby enhancing the accessibility of cellulose to cellulase enzymes and increasing the efficiency of cellulose hydrolysis, while keeping to a minimum the amount of dissolved cellulose, similarly to what occurs in a mercerization process.

In addition, NaOH and urea treatments promote the swelling and dissolution of amorphous regions of cellulose, which allows for the extraction of impurities and increases the fiber's surface area and reactivity, making it ready for enzymatic pretreatment. In other words, the invention pre-treatment step increases the surface area of the cellulosic fibers in the treated textile, especially in the yarns of a fabric, that is available for being contacted by the cellulase enzyme so that subsequent treatment with cellulase of the pre-treated yarn or fabric results in a dramatic increase of the efficiency of the enzymatic conversion of cellulose into glucose. Also, the use of the aqueous solution a temperature in the range from -25°C to +30°C, preferably 0°C to room temperature, especially when the process is carried out at room temperature, allows to reduce energy consumption and to reduce the overall costs.

In other words, the process according to the invention allows to obtain microbial cellulose, starting from waste textile materials at lower costs with respect to the known processes, but without jeopardizing the effectiveness of the process. Advantageously, the process of the invention allows to dramatically reduce the degradation and/or the dissolution of cellulose fibers during the pre-treatment step, so that an effective conversion of the cellulose into glucose is obtained. Advantageously, the process of the invention does not comprise, i.e. it does not require, a step of regeneration of solid cellulose after the pre-treatment with the aqueous solution comprising a base, preferably NaOH, and an amide, preferably urea.

Moreover, the process of the invention is particularly suitable when the waste textile includes both natural fibers (e.g., cotton fibers) and synthetic fibers (e.g., polyester fibers). Indeed, the use of an aqueous solution comprising a base, preferably NaOH, and an amide, preferably urea, at a temperature in the range from -25°C to +30°C, preferably at room temperature (20°C ± 5°C) allows to obtain a pre-treated fabric with reduced degradation of the polyester fibers (or other synthetic fibers) that can be recovered to be recycled and reused. Advantageously, the process of the invention provides an effective and possibly a complete conversion of the cellulose into glucose and allows to obtain a high yield in the production of microbial cellulose, also when the starting textile material is a waste textile material with cellulosic and non-cellulosic materials.

According to embodiments, the waste textile material comprises cellulose in the form of cellulosic fibers and non-cellulosic fibers.

In embodiments, the cellulosic fibers are virgin fibers or recycled fibers selected from plant fibers, man-made cellulosic fibers, recycled plant fibers, recycled man-made cellulosic fibers, and mixture thereof. According to embodiments, the plant fibers may be selected from cotton fibers, hemp fibers, flax (linen) fibers, jute fibers, coir fibers, sisal fibers, abaca (manila hemp) fibers, kapok fibers, ramie fibers, kenaf fibers, agave fibers, henequen fibers, lotus fibers, nettle fibers, sugar cane bagasse fibers, pineapple leaf fibers, banana tree trunk fibers, rice straw fibers and mixtures thereof. According to embodiments, the man-made cellulosic fibers may be selected from viscose fibers, lyocell fibers, modal fibers, acetate fibers, cupro fibers, carbamate fibers and mixtures thereof. In embodiments, the cellulosic fibers are selected from cotton fibers, viscose fibers and mixtures thereof.

In embodiments, the non-cellulosic fibers are selected from synthetic fibers, biosynthetic fibers, recycled synthetic fibers, recycled biosynthetic fibers and mixtures thereof.

According to embodiments, the synthetic fibers are virgin fibers or recycled fibers selected from polyester fibers, polyamide fibers, elastane fibers, polypropylene (PP) fibers, acrylic fibers, polyurethane fibers, $CO_2$-based fibers, and mixtures thereof. For example, polyester fibers may be poly(ethylene terephthalate) (PET) fibers, polytrimethylene terephthalate (PTT) fibers, poly(butylene terephthalate) (PBT) fibers, polybutylene succinate (PBS) fibers, polybutylene adipate co-terephthalate (PBAT) fibers, polylactic acid (PLA) fibers or polyhydroxyalkanoate (PHA) fibers. Recycled fibers may also be obtained by making fibers from recycled polymers, e.g. recycled polyester fibers may be obtained from recycled polyester polymer that is obtained from waste textiles, e.g. from cotton-polyester fabrics.

Biosynthetic fibers may be selected from fossil-based fibers, bio-based fibers and mixtures thereof. Fossil-based biosynthetic fibers may be selected from polylactic acid fibers (PLA) fibers, polyhydroxyalkanoate (PHA) fibers, polyurethane (PU) fibers, polytrimethylene terephthalate (PTT) fibers, polybutylene succinate (PBS) fibers, polybutylene adipate co-terephthalate (PBAT) fibers, and mixtures thereof. According to embodiments, bio-based biosynthetic fibers may be selected from bio-based polylactic acid fibers (Bio-PLA) fibers, bio-based polyhydroxyalkanoate (Bio-PHA) fibers, bio-based polyurethane (Bio-PU) fibers, bio-based polytrimethylene terephthalate (Bio-PTT) fibers, bio-based polybutylene succinate (Bio-PBS) fibers, bio-based polybutylene adipate co-terephthalate (Bio-PBAT) fibers and mixtures thereof. According to embodiments, the recycled synthetic fibers are selected from recycled polyethylene terephthalate (rPET) fibers, recycled polyamide (nylon) fibers, recycled elastane fibers, recycled polypropylene (PP) fibers, recycled acrylic fibers, recycled polyurethane (PU) fibers, recycled polytrimethylene terephthalate (PTT) fibers, polybutylene succinate (PBS) fibers and mixtures thereof.

According to embodiments, the waste textile material is selected from a garment, a fabric or a yarn, generally the process is carried out on fabrics and garments. According to embodiments, the waste textile material is a garment. For example, a fabric or a garment may be a cotton fabric or garment (i.e., a 100% cotton fabric or garment), or may comprise a combination of cotton and/or viscose fibers, possibly with polyester and/or elastane fibers. In embodiments, the waste textile is a waste textile including viscose (or cotton, or both viscose and cotton), polyester and elastane. According to embodiments, the waste textile material is a blend textile material including polyester fibers or filaments and cellulosic fibers.

According to embodiments, the waste textile material may be washed before the step of pre-treating said waste textile material with an aqueous solution comprising NaOH and urea.

In embodiments, non-textile elements such as, for example, label tags (e.g., made of leather or polyurethane), buttons, zippers (e.g., made of aluminum, brass or plastic), optionally present in the waste textile material, are removed before the step of pre-treating said waste textile material with an aqueous solution comprising NaOH and urea, optionally after washing. Removal of the non-textile elements may be carried out manually and/or with automation, using techniques and/or machines that are known, per se, in the art.

According to embodiments, the process may further comprise a step of sorting the waste textile material before or after the step of pre-treating said waste textile material with an aqueous solution comprising NaOH and urea. Preferably, the process further comprises a step of sorting the waste textile material before the step of pre-treating said waste textile material with an aqueous solution comprising NaOH and urea. In embodiments, the sorting is based on color, type of dye present in the waste textile material (e.g., indigo, sulphur dye or black reactive dye), textile (e.g., fabric) composition, weight percent cellulose, non cellulose components, or combinations thereof. For example, near infrared (NIR) sorting of textiles (e.g., fabrics and/or garments) based on color and/or textile (e.g., fabric and/or garment) composition is known in the art.

According to embodiments, the waste textile material includes a dye. When a dye is present, the process of the invention preferably includes a step of removing at least part of the dye from the waste textile material before the step of treating the textile with cellulase, preferably before the step of pre-treating said waste textile with said aqueous solution comprising NaOH and urea. Advantageously, when the dye is removed from the waste textile material before the enzymatic step with cellulase, a particularly efficient conversion of cellulose into glucose can be obtained.

According to embodiments, the dye can be removed using techniques that are, per se, known in the art. According to embodiments, the dye can be removed from the waste textile material by a method selected from ozone washing, washing with a reduction solution, extraction of the dye, e.g. using a Soxhlet extractor and a combination thereof.

According to embodiments, the dye can be removed from the waste textile material by treating the dyed waste textile material with a reducing solution including NaOH and sodium dithionite ($Na_2S_2O_4$), preferably at a temperature from 60°C to 80°C (for example, 70°C), preferably in a hermetic washing machine. According to embodiments, the concentration of NaOH in the reducing solution may be from 1 g/l to 60 g/l, preferably from 10 g/l to 30 g/l, more preferably from 15 g/l to 22.5 g/l. The concentration of sodium dithionite ($Na_2S_2O_4$) in the reducing solution may be from 1 g/l to 30 g/l, preferably from 5 g/l to 15g/l, more preferably from 7.5 g/l to 10 g/l.

According to embodiments, the process further comprises a step of reducing the size of the waste textile material, preferably after the step of pre-treating said waste textile with said aqueous solution comprising NaOH and urea. For example, the pre-treated textile material can be cut or shredded, optionally after the removal of at least part of the dye, before the step of treating said pre-treated textile with cellulase. In this case, advantageously, the contact area between the pre-treated textile material and the cellulase is increased. In embodiments, pre-treated textile as in step b) is shred into a plurality of pieces having dimension suitable for enzymatic saccharification before step c). For example, the textile obtained in step b) is shred into a plurality of pieces having average dimension from 0.5 mm$^2$ (0.005 cm$^2$) to 2500 mm$^2$ (25 cm$^2$), preferably from 1 mm$^2$ (0.01 cm$^2$) to 625 mm$^2$ (6.25 cm$^2$), more preferably from 10 mm$^2$ (0.1 cm$^2$) to 400 mm$^2$ (4 cm$^2$), e.g. an area of 100 mm$^2$ before step c).

Additionally, pre-treating the waste textile material before shredding results in a reduced amount of dissolved cellulose in pre-treatment step, i.e., in a reduction of the amount of cellulose that is dissolved during the pre-treatment with the aqueous solution comprising NaOH and urea without jeopardizing the enzymatic conversion of cellulose into glucose. Moreover, when the waste textile material includes non-cellulosic fibers, such as polyester fibers, cutting or shredding the textile material after the pre-treatment step allows to reduce the amount of non-cellulosic material (e.g., polyester) that could be lost during the cut or shredding. Also, when the waste textile material includes non-cellulosic fibers, such as polyester fibers, cutting or shredding the textile material after the pre-treatment step allows to reduce or avoid possible detrimental effects of the NaOH/urea solution on the non-cellulosic material (e.g., polyester). Advantageously, cutting or shredding the textile material after the pre-treatment step allows to recover a greater amount of non-cellulosic materials (e.g., polyester fibers) with respect to cutting or shredding the textile material before the pre-treatment step with the aqueous solution comprising NaOH and urea because a part of polyester particles will be lost with the NaOH /urea solution.

Shredding may be carried out mechanically, as known in the art. In an embodiment the waste textiles are transferred into a thermally insulated container and liquid nitrogen is poured onto the waste textiles. The thus obtained frozen waste textiles are milled until small pieces between 1 mm - 5mm are obtained. Preferably, average size is 1 mm. The reduced size particles are then treated following the invention process steps such as cellulase treatment. Advantageously, the size of the fabric to be pre-treated may be quite large, e.g. a piece of fabric large about 180 cm and long 4-6 meters. Similarly, garments from which non-textile elements have been removed may be first pre-treated and subsequently shredded only after pre-treatment with NaOH/urea.

As previously mentioned, the step of pre-treating the waste textile material with an aqueous solution comprising NaOH and urea provides improved results, namely time and efficiency, of the enzymatic conversion of the fabric cellulose into glucose with cellulase. Without being bound to a specific scientific explanation, it has been observed that the invention pre-treatment of the waste textile material with an aqueous solution comprising NaOH and urea at a temperature ranging from -25°C to +30°C, preferably +10°C to +25°C, more preferably room temperature of +15°C to +25°C, provides for a swelling of the textile material, so that an effective conversion of cellulose into glucose, using cellulase enzymes, can be obtained. In other words, it has been observed that the pre-treatment of the invention results in a separation of the fibers of the textile (e.g., the distance between the fibers increases) in an effect also known as swelling, so that the cellulase enzyme can effectively reach the cellulose chains, whereby an effective conversion of cellulose into glucose is obtained.

According to embodiments, the aqueous solution comprising NaOH and urea comprises NaOH in an amount ranging from 1% to 15% w/v, preferably from 2% to 10% w/v, more preferably from 3% to 9% w/v. For example, the aqueous solution comprising NaOH and urea may comprise the NaOH in an amount ranging from 6% to 8% w/v, for example 7% w/v.

According to embodiments, the aqueous solution comprising NaOH and urea comprises the urea in an amount ranging from 1% to 25% w/v, preferably from 3% to 20% w/v, more preferably from 5% to 15% w/v. For example, the aqueous solution comprising NaOH and urea may comprise the urea in an amount ranging from 11% w/v to 13% w/v, for example 12% w/v.

According to embodiments, in the step of pre-treating said waste textile material with said aqueous solution comprising NaOH and urea, the waste textile material is in an amount in the range from 1% w/v from 20% w/v, preferably from 2% w/v to 15% w/v, more preferably from 2.5% w/v to 10% w/v with respect to the volume of said aqueous solution comprising NaOH and urea. For example, the waste textile material may be in an amount in the range from 3% w/v from 7% w/v, preferably from 3.5% w/v to 6.5% w/v, more preferably from 4% w/v to 6% w/v, for example 5% w/v, with respect to the volume of said aqueous solution comprising NaOH and urea.

According to embodiments, the step of pre-treating said waste textile material with said aqueous solution comprising NaOH and urea is carried out at a temperature ranging from -15°C to +25°C, preferably at room temperature i.e. from +15°C to +25°C.

According to embodiments, the step of pre-treating said waste textile material with said aqueous solution comprising NaOH and urea has a duration in the range from 0.5 hours to 10 hours, preferably from 1 hour to 8 hours, more preferably from 2 hours to 7 hours. For example, the step of pre-treating said waste textile material with said aqueous solution comprising NaOH and urea may have a duration from 4 hours to 6 hours, for example 5 hours.

For example, the step of pre-treating said waste textile material may be carried out using an aqueous solution comprising NaOH and urea that comprises NaOH in an amount in the range from 3% to 9% w/v, preferably from 6% to 8% w/v, , urea in an amount in the range from 5% to 15% w/v, preferably from 11% to 13% w/v, at a temperature ranging from -15°C to +25°C, preferably at room temperature, for 1 hour to 8 hours, more preferably 2 hours to 7 hours. In an embodiment, a pre-treating solution containing about 7% w/v NaOH, 12% w/v urea is used at room temperature for 2 hours to provide the required separation of the fibers (e.g., by swelling of the fibers) of a blend fabric (5% w/v) cotton/polyester.

According to embodiments, the pre-treated textile obtained after the step of pre-treating with the aqueous solution comprising NaOH and urea is washed. The washing can be carried out, for example with a weak acid solution (e.g., an acetic acid solution) or with water. The water may be, for example, tap water, distilled water, reverse osmosis water, or a mixture thereof. The washing is preferably carried out at a temperature ranging from 10°C to 30°C, preferably from 15°C to 28°C, for example 25°C. According to embodiments, after the washing step, the pH of the aqueous extract of the pre-treated textiles in the range from 6 to 8, preferably from 6.5 to 7.5, for example 7. The pH of the aqueous extract of the pre-treated textile is measure according to ISO 3071:2020 standard.

According to embodiments, the textile obtained after the step of pre-treating with the aqueous solution comprising NaOH and urea and, optionally, after washing, is dried. For example, the drying is preferably carried out at a temperature ranging from 50°C to 100°C, preferably from 70°C to 90°C, for example 90°C in industrial drying machines known per se in the art such as tumble dryers or conveyor belt dryers. According to embodiments, the textile obtained after the step of pre-treating with the aqueous solution comprising NaOH and urea, optionally after washing and, optionally after drying, is cut or shredded (e.g., into small pieces or fragments), to reduce the size of the pre-treated textile material. For example, the textile obtained after the step of pre-treating with the aqueous solution comprising NaOH and urea is cut or shred into a plurality of pieces having average dimension (area) from 0.5 mm$^2$ (0.005 cm$^2$) to 2500 mm$^2$ (25 cm$^2$), preferably from 1 mm$^2$ (0.01 cm$^2$) to 625 mm$^2$ (6.25 cm$^2$), more preferably from 10 mm$^2$ (0.1 cm$^2$) to 400 mm$^2$ (4 cm$^2$), e.g. 100mm$^2$ (1 cm$^2$ e.g. 1×1 cm).

According to embodiments, after the step of pre-treating the textile with the aqueous solution comprising NaOH (or another base) and urea (or another amide), the aqueous solution comprising NaOH (or another base) and urea (or another amide) can be reused. According to embodiments, the aqueous solution comprising NaOH (on another base) and urea (or another amide) can be reused from 1 to 20 times, preferably 5 to 15 times, more preferably 8 to 12 times, for example 10 times. In embodiments, the concentration of NaOH (or another base) and urea (or another amide) can be adjusted, if necessary, before being reused. For example, if necessary, NaOH concentration can be adjusted to a range from 1% to 15% w/v, preferably from 2% to 10% w/v, more preferably from 3% to 9% w/v. For example, if necessary, urea concentration can be adjusted to a range from 1% to 25% w/v, preferably from 3% to 20% w/v, more preferably from 5% to 15% w/v.

According to embodiments, the step of pre-treating said waste textile material with said aqueous solution comprising NaOH and urea is carried out in a reaction chamber; wherein the aqueous solution comprising NaOH and urea is provided to the waste textile material in the reaction chamber, and circulated through said waste textiles. Preferably, the waste textiles are in a still, i.e. static, condition and the solution comprising NaOH and urea is circulated through them to provide pre-treated textiles. When the solution aqueous solution comprising NaOH and urea is circulated through said waste textiles, the duration of the pre-treatment can be particularly short.

The term "circulated" referred to the aqueous solution comprising NaOH (or another base) and urea (or another amide) is intended to define that the solution is subjected to a flow that is forced through the textiles and through a reaction chamber in which the textiles are held. The flow is generated preferably by a pump that feeds the solution into the reaction chamber through the textiles and through the chamber. The solution leaves the reaction chamber and is subsequently fed back to it to be circulated again through textiles and through the chamber. According to embodiments, when the pre-treated textile obtained after the step of pre-treating with the aqueous solution comprising NaOH and urea is washed, the washing solutions can be also circulated through the textiles several times in the same way as the solution comprising NaOH and urea. In embodiments, the plant for carrying out the step of pre-treating said waste textile material with said aqueous solution comprising NaOH and urea comprises a fluidic circuit typically including an inlet and an outlet to the reaction chamber. Preferably in this embodiment the bath ratio, i.e. weight of fabric to volume of solution is 5% w/v, with respect to the volume of said aqueous solution comprising NaOH and urea

The term "still" or "static" condition is intended to define that the textiles are housed in a reaction chamber and are held there while the aqueous solution comprising NaOH and urea (or the washing solution) is fed through the textiles. The textiles fill the chamber so that they do not move with the solution, i.e. they are not displaced under the action of the flow of solution. In embodiments, the textiles are compressed in the chamber.

According to an aspect, the process of the invention further includes a step of treating with cellulase the textile material obtained by pre-treating it with the aqueous solution comprising NaOH and urea, in order to convert the cellulose present in the textile into glucose.

According to embodiments, the pre-treated textile material is treated with a solution comprising cellulase, i.e., cellulase enzymes.

According to embodiments, the amount by weight of the pre-treated textile material with respect to the volume of solution comprising cellulase is in the range from 1% weight volume (w/v) to 20% w/v, preferably from 2% w/v to 15% w/v, more preferably from 2.5% w/v to 10% w/v. For example, 5 gr of pre-treated waste textile may be treated with 100 ml of solution comprising cellulase: in this case, the pre-treated textile material is in an amount in of 5% w/v with respect to the volume of solution comprising cellulase.

According to embodiments, the cellulase is in an amount so that the enzymatic activity is from 1 FPU (Filter Paper Unit) per gram of cellulose to 50 FPU per gram of cellulose, preferably from 10 FPU per gram of cellulose to 30 FPU per gram of cellulose, more preferably from 15 FPU per gram of cellulose to 25 FPU per gram of cellulose. The enzymatic activity of the cellulase can be measured according to methods that are known, per se, in the art. For example, the enzymatic activity of the cellulase can be measured according to Adney B. and Baker J., "Measurement of cellulase activity", National Renewable Energy Laboratory, 1996; or Selig M., Weiss N., and Ji Y., "Enzymatic saccharification of lignocellulosic biomass", National Renewable Energy Laboratory, 2008. Cellulase suitable to be used according to the invention are known, per se, in the art. For example, suitable currently commercially available products Novozymes Cellic® CTec3 HS, AB Enzyme - Flashzyme Plus 200, Novozymes Cellic®CTec2 and Novozymes Cellusoft®AB Cone can be used.

According to embodiments, the enzymatic step of treating the pre-treated textile material with cellulase is carried out at a temperature ranging from 30°C to 70°C, preferably from 40°C to 60°C, more preferably from 45°C to 55°C. For example, the step of treating the pre-treated textile material with cellulase may be carried out at a temperature of 50°C.

According to embodiments, the step of treating the pre-treated textile material with cellulase is carried out for a time in the range from, 10 min to 144 hours, preferably from 30 min to 96 hours, more preferably from 12 hour to 72 hours, even more preferably from 18 hours to 48 hours, still more preferably from 20 hours to 36 hours. For example, the step of treating the pre-treated textile material with cellulase may be carried out for 24 hours.

According to embodiments, the step of treating the pre-treated textile material with cellulase may be carried out under stirring at a speed in the range from 5 rpm (revolutions per minute) to 200 rpm, for example between 50 rpm and 100 rpm. Preferably, the enzymatic step of treating the pre-treated textile material with cellulase is carried out with no agitation (i.e., no stirring).

According to embodiments, the step of treating the pre-treated textile material with cellulase is carried out at a pH in the range from 3.0 to 6.0, preferably from 4.0 to 5.0. For example, the step of treating the pre-treated textile material with cellulase may be carried out at pH 5.0.

According to embodiments, the pre-treated textile material includes a dye and at least part of the dye is removed from the pre-treated textile material. In other words, in embodiments, at least part of the dye can be removed after the step of pre-treating the waste textile material with an aqueous solution comprising NaOH and urea, before the step of treating the pre-treated textile material with cellulase.

In preferred embodiments, the pre-treated textile material is not dyed. In other words, in embodiments, the step of treating the pre-treated textile material with cellulase is carried out to pre-treated textile materials that are not dyed.

For example, the step of treating the pre-treated textile material with cellulase may be carried out using an aqueous solution comprising cellulase enzymes in an amount ranging from 1 FPU (Filter Paper Unit) per gram of cellulose to 50 FPU per gram of cellulose, preferably from 10 FPU per gram of cellulose to 30 FPU per gram of cellulose, more preferably from 15 FPU per gram of cellulose to 25 FPU per gram of cellulose, at a temperature ranging from 30°C to 70°C, preferably from 40°C to 60°C, more preferably from 45°C to 55°C, for 10 min to 144 hours, preferably for 30 min to 96 hours, more preferably from 12 hour to 72 hours, even more preferably from 18 hours to 48 hours, still more preferably from 20 hours to 36 hours, with no agitation.

According to embodiments, at the end of the step of treating the pre-treated textile material with cellulase a mixture including a solid phase and a liquid phase is obtained. For example, the solid phase may include non-cellulosic fibers (e.g., polyester fibers) that were included in the waste textile material and, optionally, non-soluble dyes (such as indigo dye and sulphur dye), if present, and optionally, undigested cellulosic fibers and materials. For example, the liquid phase may include the cellulase solution used for the treatment of the pre-treated textile material, glucose and, optionally, soluble dyes (such as reactive black dye), if present.

According to an aspect, the process of the invention includes a step of separating said liquid phase from said solid phase, to obtain a liquid mixture including glucose.

According to embodiments, when the waste textile material includes non-cellulosic fibers, said non-cellulosic fibers are recovered from said solid phase of said mixture including a solid phase and a liquid phase, for example after said separating step.

According to embodiments, the separation of the liquid phase from the solid phase may be carried out by gravity, preferably waiting for heavy materials (polyester, elastane) to settle at the bottom of the reactor, then discharging supernatant liquid phase. Additionally, or alternatively, the separation of the liquid phase from the solid phase may include a step of filtration or multi-stage filtration (also known as sequential cascade filtration), preferably via sieve having pore size in the range from 0.3 mm to 0.5 cm, via centrifugation, preferably at a speed in the range from 5000 rpm (revolutions per minute) to 50000 rpm, or a combination thereof. For example, in embodiments, the mixture including a solid phase and a liquid phase may be filtered using a sieve, preferably having pore size of 0.3 mm. In embodiments, the mixture including a solid phase and a liquid phase, or the liquid phase can be centrifuged, for example at 9000 rpm for 30 minutes. The supernatant obtained after centrifugation may be recovered as the liquid mixture including glucose. In other words, the liquid phase obtained after the separation step, e.g., after the separation of the liquid phase from the solid phase (e.g., by filtration or multi-stage filtration, or via centrifugation, or using a combination thereof), can be recovered as the liquid mixture including glucose. Suitable separators are clarifiers, particularly clarifiers for food ingredients.

According to embodiments, the cellulase enzymes can be recovered from the liquid phase and, preferably, reused. Cellulase enzymes may be recovered using techniques that are known, per se in the art, such as, for example, dialysis or ultrafiltration. According to embodiments, the recovered cellulase enzymes can be reused from 1 to 10 times, preferably 2 to 8 times, more preferably 2 to 5 times, for example 3 times. In embodiments, the amount of cellulase can be adjusted, if necessary. For example, if necessary, cellulase amount can be adjusted for reuse, so that the enzymatic activity is from 1 FPU (Filter Paper Unit) per gram of cellulose to 50 FPU per gram of cellulose, preferably from 10 FPU per gram of cellulose to 30 FPU per gram of cellulose, more preferably from 15 FPU per gram of cellulose to 25 FPU per gram of cellulose.

According to embodiments, the cellulase enzymes may be recovered and reused for treating the solid phase obtained after the separation step and/or to treat one or more additional pre-treated waste textile materials.

According to embodiments, the cellulase enzymes may be recovered and reused, for example from 1 to 10 times, preferably 2 to 8 times, more preferably 2 to 5 times, for example 3 times, to treat the solid phase obtained after the separation step. Reusing the cellulase enzymes to treat the solid phase obtained after the separation step allows to digest the cellulosic fibers and materials that were not digested in the previous treatment or treatments with cellulase, thus increasing the yield of the conversion of cellulose to glucose. The enzymatic treatment of the solid phase results in an additional amount of mixture including a solid phase and a liquid phase, according to the above.

According to embodiments, the cellulase enzymes may be recovered and reused, for example from 1 to 10 times, preferably 2 to 8 times, more preferably 2 to 5 times, for example 3 times, to treat one or more additional pre-treated waste textile materials.

According to embodiments, at least part of the dye may be removed and/or recovered from said solid phase or from said liquid phase of said mixture including a solid phase and a liquid phase, preferably after said step of separating said liquid phase from said solid phase. For example, if the dye is to be removed and/or recovered from the liquid phase, the dye can be removed from the liquid phase by passing the liquid phase through an activated carbon column.

According to an aspect, the process of the invention includes a step of preparing a microbial culture using the mixture including glucose, preferably obtained after the separating step, wherein the microbial culture comprises microbial cellulose-producing microorganisms.

According to embodiments, the step of preparing a microbial culture using the mixture including glucose includes, optionally, a step of concentrating the mixture including glucose to increase the concentration of said glucose. Advantageously, by concentrating the mixture including glucose, the volume of the mixture is reduced, so that the handling and, optionally, the storage of the mixture is easier. According to embodiments, the concentrating step may be carried out using a heat exchanger, a rotary evaporator, a vacuum evaporator, or a combination thereof. For example, the concentration of glucose in the mixture including glucose may be increased by treating the mixture including glucose in a heat exchanger, e.g., at 100°C for a time ranging from 1 hour to 12 hours, optionally depending on the volume of the mixture including glucose to be concentrated and/or the final glucose concentration to be obtained.

According to embodiments, the amount of the glucose in a mixture including glucose can be measured using the 3,5-dinitrosalicylic acid (DNS) method. The DNS method is a method that is, per se, known in the art. The DNS method is disclosed, for example in Ghose, T.K., 1987, "Measurement of Cellulase Activities", Pure & Applied Chemistry 59:257-268; and in Miller G.L., 1959, "Use of Dinitrosalicylic Acid Reagent for Determination of Reducing Sugar", Analytical Chemistry 31:426-428.

In embodiments, the percentage of the cellulose converted into glucose is determined. Preferably, the percentage of the cellulose converted into glucose is calculated using the following formula:

$$Cellulose\ Conversion\ (\%) = \frac{Glucose\ released\ from\ enzymatic\ hydrolysis\ (g/L) \times Reaction\ volume\ (L)}{Substrate\ weight\ (g) \times Cellulose\ content\ (\%)} \times 0.9 \times 100\%$$

where: the "glucose released from enzymatic solution" is determined using the DNS method; the "reaction volume" is the volume of the solution including cellulase used for treating the pre-treated textile; the "substrate weight" is the initial weight of the waste textile material; the "cellulose content" is the amount of cellulose in the waste textile material expressed as percentage of the initial weight of the waste textile material, and "0.9" is a correction factor for water molecules added upon hydrolysis of the cellulose.

According to embodiments, the step of preparing a microbial culture using said mixture including glucose includes a step of adjusting the concentration of the glucose in said mixture including glucose to a concentration from 10 g/L to 30 g/L, preferably from 15 g/l to 25 g/l. According to embodiments, includes a step of adjusting the concentration of the glucose in said mixture including glucose may be carried out by concentrating the mixture including glucose, by diluting the mixture including glucose or by a combination thereof. For example, the mixture including glucose may be concentrated and, after the measurement of the amount of glucose in the concentrated mixture including glucose, diluted to obtain the required concentration of glucose, e.g., a concentration of glucose from 10 g/L to 30 g/L, preferably from 15 g/l to 25 g/L. In embodiments, the mixture including glucose, without being concentrated, may be diluted to obtain the required concentration. In embodiments, if the cellulases are removed from the liquid phase obtained after the separating step by means of dialysis, a reduction in the concentration of the glucose in the mixture including glucose is obtained; in this case, the concentration of the glucose in the mixture could be adjusted to obtain the required concentration of glucose, e.g., a concentration of glucose from 10 g/L to 30 g/L, preferably from 15 g/l to 25 g/L.

According to embodiments, the mixture including glucose is sterilized, preferably in an autoclave. In this case, advantageously, the enzymes that may be present in the mixture are deactivated and can be used as source of amino acids for microbial cellulose-producing microorganisms. In embodiments, the cellulase enzymes are not removed from the liquid phase obtained after the separating step. In embodiments, the concentration of amino acids in the mixture can be adjusted, if necessary. For example, the concentration of amino acids in the mixture can be adjusted by adding peptone to the mixture. For example, if enzymes are recovered and reused for treating additional pre-treated textiles, then additional nitrogen and carbon source (such as peptone) may be added. Indeed, the enzymatic treatment of each additional textile results in more glucose in the mixture, resulting in a not sufficient amount of amino acids in the mixture including glucose. In embodiments, if enzymes are recovered and reused for treating additional pre-treated textiles, the cellulase enzymes are not removed from the liquid phase obtained after the separating step carried out after the enzymatic treatment of the last textile. In embodiments, if the cellulase enzymes are recovered and reused for treating the solid phase obtained after the separation step (i.e., second enzymatic treatment carried out on the solid phase obtained from a single starting waste textile material), then additional nitrogen and carbon source (such as peptone) may be not necessary. In embodiments, if the cellulase enzymes are recovered and reused for treating the solid phase obtained after the separation step (i.e., second enzymatic treatment carried out on the solid phase obtained from a single starting waste textile material), the cellulase enzymes are not removed from the liquid phase obtained after the last separating step carried out after the last enzymatic treatment.

According to an aspect, the mixture including glucose, preferably having a concentration of the glucose in said mixture from 10 g/L to 30 g/L, preferably from 15 g/l to 25 g/l, for example 20 g/l, can be used as "waste textile glucose" (WTG) medium for the preparation of the microbial culture.

According to embodiments, the step of preparing a microbial culture using the mixture including glucose includes a step of mixing the mixture including glucose with a starter microbial culture comprising microbial cellulose-producing microorganisms.

According to embodiments, the amount of said mixture including glucose in the microbial culture is from 99% to 80% by weight, preferably from 95% to 85% by weight of the microbial culture weight.

According to embodiments, the amount of the starter microbial culture comprising microbial cellulose-producing microorganisms in the microbial culture is from 1% to 20% by weight, preferably from 5% to 15% by weight of the microbial culture weight.

As used herein, the term "starter microbial culture" refers to a culture of microorganisms that is used, usually in small amount, to inoculate a batch of medium in order to ferment it. A starter culture is, usually, a microbiological culture which actually performs fermentation, that is subsequently added to a medium to start the fermentation process.

According to embodiments, the step of preparing a microbial culture using said mixture including glucose comprises a step of adding at least one additive to said mixture including glucose, wherein said additive is preferably selected from titanium (IV) bis-(ammonium lactate) dihydroxide (TiBALDH), glycerol, ascorbic acid, ethephon (2-chloroethylphosphonic acid) and mixtures thereof.

Advantageously, when one or more additives are used, the amount of microbial cellulose (e.g., bacterial cellulose) that is obtained is increased with respect to the amount obtained without using additives.

According to embodiments, the additive is titanium (IV) bis-(ammonium lactate) dihydroxide (TiBALDH), preferably in an amount from 0.0005% to 1% (w/v), more preferably from 0.0025% to 0.4% (w/v), even more preferably in an amount from 0.05%% to 0.3% (w/v), for example 0.25% (w/v).

According to embodiments, the additive is glycerol, preferably in an amount from 0.1% to 10% (w/v), more preferably from 0.5% to 2% (w/v), even more preferably in an amount from 0.75% to 1.5% (w/v), for example 1% (w/v).

According to embodiments, the additive is ascorbic acid, preferably in an amount from 0.05% to 10% (w/v), more preferably from 0.25% to 2% (w/v), even more preferably in an amount from 0.3% to 1% (w/v), for example 0.5% (w/v).

According to embodiments, the additive is ethephon (2-chloroethylphosphoric acid), preferably in an amount from 0.001mM to 10 mM, more preferably from 0.005 mM to 2 mM, even more preferably in an amount from 0.01 mM to 1 mM, for example 0.01 mM.

According to embodiments, the additive is a mixture of glycerol and titanium (IV) bis-(ammonium lactate) dihydroxide (TiBALDH), wherein said glycerol is added to said mixture including glucose preferably in and amount from 0.1% to 10% (w/v), more preferably between 0.5% to 2% (w/v), even more preferably in an amount of 1% (w/v), and wherein said titanium (IV) bis-(ammonium lactate) dihydroxide (TiBALDH) is added to said mixture including glucose preferably in and amount from 0.0005% to 1% (w/v), more preferably between 0.0025% to 0.4% (w/v), even more preferably in an amount of 0.25% (w/v).

As used herein, the term "microorganism" refers to an organism that is too small to be seen with naked eye. As used herein, the term "microorganism" encompasses not genetically modified (i.e. wild type) microorganisms and genetically modified microorganism as well.

According to embodiments, the microbial cellulose-producing microorganisms are selected from bacteria, algae, yeast and mixtures thereof. According to embodiments, the microbial cellulose-producing microorganisms may be genetically modified microorganisms. In preferred embodiments, the microbial cellulose producing microorganisms are bacteria.

In embodiments, the microbial cellulose can be selected microbial cellulose produced by bacteria (i.e., bacterial cellulose), microbial cellulose produced by algae, microbial cellulose produced by yeasts, and mixtures thereof. According to embodiments, the microbial cellulose can be selected from microbial cellulose produced by bacteria (i.e., bacterial cellulose), microbial cellulose produced by algae, and mixtures thereof. Preferably, the microbial cellulose is bacterial cellulose.

According to embodiments, microbial cellulose-producing bacteria are selected from *Gluconacetobacter*, genetically modifed *Gluconacetobacter*, *Komagateibacter*, genetically modified *Komagateibacter*, *Aerobacter*, genetically modified *Aerobacter*, *Acetobacter*, genetically modified *Acetobacter*, *Achromobacter*, genetically modified *Achromobacter*, *Agrobacterium*, genetically modified *Agrobacterium*, *Azotobacter*, genetically modified *Azotobacter*, *Salmonella*, genetically modified *Salmonella*, *Alcaligenes*, genetically modified *Alcaligenes*, *Pseudomonas*, genetically modified *Pseudomonas*, *Rhizobium*, genetically modified *Rhizobium*, *Sarcina*, genetically modified *Sarcina*, genetically modified *Enterobacter*, *Escherichia*, genetically modified *Escherichia*, *Bacillus*, genetically modified *Bacillus* genus, *Klebsiella*, genetically modified *Klebsiella*, and mixtures thereof. According to preferred embodiments, microbial cellulose-producing bacteria are selected from *Gluconacetobacter hansenii*, *Gluconacetobacter xylinus*, *Komagateibacter xylinus* and mixtures thereof. According to preferred embodiments, bacterial cellulose-producing bacteria are selected from *Gluconacetobacter hansenii* ATCC 53582, *Gluconacetobacter xylinus* ATCC 23770, *Komagateibacter xylinus* DSM46604, and mixtures thereof. For example, the bacterial-cellulose producing bacteria may be *Gluconacetobacter hansenii* ATCC 53582 bacteria.

According to embodiments, microbial cellulose-producing algae are selected from *Phaeophyta*, *Chlorophyta*, *Rhodophyta*, *Ochrophyta*, *Ascophyllum*, *Chlorella*, *Nannochloropsis*, *Ulva*, *Cladophora*, *Valonia* and mixtures thereof. According to embodiments, microbial cellulose-producing algae are selected from *Ascopyllum nodosum*, *Chlorella vulgaris*, *Nannochloropsis gaditana*, *Ulva prolifera*, *Ulva pertusa*, *Cladophora glomerata*, *Valonia ventricosa*, and mixtures thereof. It is known in the art that heterotrophic and mixotrophic cultivation of algae under dark condition requires glucose as a carbon source.

Cellulose-producing yeasts are known e.g. from Jasme, Nurshafqah et al. (2022) First report of biocellulose production by an indigenous yeast, Pichia kudriavzevii USM-YBP2. Green Processing and Synthesis. Vol 11 issue 1.

According to an aspect, the process of the invention includes a step of incubating the microbial culture to obtain microbial cellulose. In embodiments, a bacterial culture is incubated to obtain bacterial cellulose.

According to embodiments, the incubating step is carried out at a temperature in the range from 15°C to 35°C, preferably from 20°C to 30°C. For example, the incubating step may be carried out at a temperature from 25°C to 30°C, for example 28°C.

According to embodiments, the incubating step may have a duration in the range from 1 to 30 days, preferably 5 to 20 days, more preferably from 10 to 16 days. For example, the incubating step may have a duration from 13 to 15 days, for example 14 days.

In embodiments, the starter culture medium may be Hestrin Schramm (HS) Medium, and may have, for example, the following composition:
20.0 gr/L Glucose; 5.0 gr/L Peptone from soybean, enzymatic digest; 5.0 gr/L Yeast extract; 3.4 gr/L di-Sodium hydrogen phosphate dihydrate; 1.5 gr/L Citric acid monohydrate; 1.0 L Distilled water.

In embodiments, the mixture including glucose, before the addition of the starting culture, may have, for example, the following composition:
20.0 gr/L Glucose, derived from the enzymatic degradation of the cellulose in the waste textile material; 2.85 mL/L Peptone, derived from degraded cellulase enzymes; 10.5 gr/L Citric acid, derived from citric acid buffer solution used in the treatment with cellulase enzymes; 1.0 L Used water, derived from the mixture used in the treatment with cellulase enzymes.

According to embodiments, the mixture including glucose and the starter culture may be admixed in a volume ratio mixture including glucose: starter culture from 99:1 to 80:20, preferably from 95:5 to 85:15, for example 90:10.

The culture comprising microbial cellulose-producing microorganisms may be, for example, incubated in one or more sterile containers. The containers may have a cover or not have a cover. Preferably, the containers have a cover. For example, the containers may have a cover that acts as a physical barrier (i.e., a barrier which has no air permeability e.g., a plastic lid) or acts as a partial physical barrier (for example, a spunbond nonwoven layer, having high air permeability, or a meltblown nonwoven layer, having low air permeability).

The incubation may be carried out at a temperature in the range from 15°C to 35°C, preferably from 20°C to 30°C, for from 1 to 30 days, preferably from 5 to 20 days, more preferably from 10 to 16 days. For example, the incubating step may be carried out at a temperature from 25°C to 30°C, for example of 28°C, for from 13 to 15 days, for example of 14 days.

According to embodiments, the obtained microbial cellulose (e.g., bacterial cellulose) is washed and, optionally, dried.

According to embodiments, the washing is carried out using an aqueous solution including a bleaching agent.

In embodiments, the bleaching agent can be selected from NaOCl, NaOH, H$_2$O$_2$ or mixtures thereof. In preferred embodiments, the bleaching agent is NaOCl. According to embodiments, the washing is carried out using an aqueous solution including NaOCl in an amount in the range from 0.01% to 10% (w/v), preferably in an amount in the range from 0.1% to 0.05% (w/v).

According to embodiments, the liquor ratio by weight of microbial cellulose layers/washing solution is 1/10, preferably 1/4.

According to embodiments, the washing is carried out using an aqueous solution including NaOH in an amount in the range from 0.01% to 10% (w/v), preferably in an amount in the range from 1% to 5% (w/v).

According to embodiments, the washing is carried out using an aqueous solution including H$_2$O$_2$ in an amount in the range from 0.01% to 10% (w/v), preferably in an amount in the range from 1% to 5% (w/v).

According to embodiments, the step of washing the microbial cellulose (e.g., bacterial cellulose) is carried out at a temperature ranging from 20°C to 80°C, preferably from 25°C to 50°C, more preferably from 25°C to 35°C. For the step of washing the bacterial cellulose is carried out at a temperature of 30±2°C. According to embodiments, the step of washing the microbial cellulose is carried out on microbial cellulose in a laundry bag. For example, the laundry bag may be made of a fabric having mesh size between 50 micrometers and 100 micrometers, preferably 75 micrometers.

According to embodiments, the step of washing the microbial cellulose (e.g. bacterial cellulose) may be carried out under stirring from 5 rpm (revolutions per minute) to 250 rpm, preferably between 50 rpm and 75 rpm. For example, the step of washing the microbial cellulose (e.g. bacterial cellulose) may be carried out under stirring at 100 rpm.

Advantageously, the process of the invention allows to obtain a microbial cellulose, particularly a bacterial cellulose, that has low content of inorganic compounds. Advantageously, the content of inorganic materials in the microbial cellulose of the invention is less than 1% by weight measured according to ISO 1762:2019, determination of residue (ash content) on ignition at 525°C.

As above discussed, one advantage of the invention process is that in a blend textile, e.g. a blend fabric, the process results in minimizing the possible damage to the polyester parts of the blend textile, i.e. degradation of the polyester filaments or fibers is kept to a minimum in order to recover polyester material for being recycled.

The present invention relates, thus, also to a process of recycling blend textiles that include polyester fibers or filaments and cellulosic fibers, comprising the following steps:

g) providing a waste textile material;

h) pre-treating said waste textile material with an aqueous solution comprising NaOH and urea, to obtain a pre-treated textile material, wherein said step of pre-treating said waste textile material with an aqueous solution comprising NaOH and urea is carried out at a temperature in the range from -25°C to +30°C;

i) treating said pre-treated textile material with cellulase, to convert said cellulose into glucose, whereby a mixture including a solid phase including polyester fibers or filaments and a liquid phase is obtained;

j) separating said liquid phase from said solid phase, to obtain a liquid mixture including glucose,

k) recovering said polyester fibers or filaments from said solid phase.

All the features herein discussed with reference to the process for the production of microbial cellulose from a waste textile material, and with reference to the process of enzymatic saccharification of a waste textile material, also apply to the process of recycling blend textiles that include polyester fibers or filaments and cellulosic fibers, above mentioned.

Figure 1 schematically summarizes an exemplary embodiment of the process of the invention, as follows.

The waste textile material is collected and, optionally, washed for cleaning purposes (block 1 of figure 1). The waste textile material comprises cellulose, e.g., natural cellulosic fibers such as cotton fibers and/or regenerated cellulose fibers such as viscose fibers, and can, optionally, further comprise non-cellulosic fibers, such as polyester and/or elastane. For example, the waste textile material may be selected from yarns, fabrics, garments and combination thereof.

After the collection and the washing of the waste textile material, if necessary, non textile elements such as, for example, label tags (e.g., made of leather or polyurethane), buttons and zippers (e.g., generally made of aluminum, brass or plastic) are removed manually or automatically (using machines and devices that are, per se, known in the art) from the waste textile material (block 2 of figure 1).

The waste textile material is, then, sorted, for example, in view of the color, the type of dye present in the waste textile material (e.g., indigo, sulphur dye or black reactive dye) or the textile (e.g., fabric) composition (e.g., 100% cotton textiles may be separated from textile containing both cotton and polyester fibers). For example, near infrared (NIR) sorting of textiles (e.g., fabrics and/or garments) based on color and/or textile (e.g., fabric and/or garment) compositions is known in the art (block 3 of figure 1).

If the waste textile material includes one or more dyes, the dyes are preferably removed, for example, by treating the fabric with a reducing solution of NaOH and sodium dithionite (Na$_2$S$_2$O$_4$) (block 4 of figure 1).

The waste textile material is pre-treated (block 5) with an aqueous solution comprising NaOH and urea, at a temperature in the range from -25°C to +30°C to swell the yarns and fibrillate the cellulose fibers. The step of pre-treating may be carried out for different times, depending on the temperature and concentration of the pre-treating solution. Typical treatment times are from 1 hour to 8 hours, more preferably from 2 hours to 5 hours. The treated textile material may be washed, for example with a weak acid solution (e.g., an acetic acid solution) or with water (block 5 of figure 1). The pre-treated textile material, preferably after washing, may be dried. Pre-treated waste textiles are then, preferably, shredded to reduce the size of the waste textile materials and increase the surface area that can be contacted with a solution comprising cellulase enzymes (block 5a in Figure 1). The aqueous solution comprising NaOH and urea may be, optionally, reused, e.g. in pre-treating further waste textile materials, up to 20 times; the concentration of NaOH and/or urea may be adjusted for reuse, if necessary.

After the pre-treatment and washing steps, the thus obtained textile material, preferably dry and shredded, is contacted with a solution comprising cellulase enzymes, to convert the cellulosic fibers of the textile material into glucose (block 6). Preferably, the step of treating the pre-treated textile material with cellulase is carried out with no agitation at a pH in the range from 4.0 to 5.0. At the end of the step of enzymatic conversion of cellulosic fibers into glucose a mixture including a solid phase and a liquid phase is obtained (block 6 of figure 1).

The solid phase will include non-cellulosic fibers (e.g., polyester fibers and/or elastane fibers), undigested cellulosic fibers and non-soluble dyes (such as indigo dye and sulphur dye), if dyes are still present. The liquid phase typically includes the cellulase solution used for the treatment of the pre-treated textile material, glucose and, optionally, soluble dyes (such as reactive black dye), if present.

After the step of treating the pre-treated textile material with cellulase, the obtained mixture is treated to separate the liquid phase from the solid phase (block 7 of figure 1). At the end of the separating step, a liquid mixture including glucose is obtained (block 9 of figure 1); this liquid mixture contains the cellulase enzymes that were added in the previous step. In embodiments, the cellulase enzymes may be recovered and, optionally, reused for treating the solid phase obtained after the separation step, up to 10 times (not shown in Figure 1). In embodiments, the cellulase enzymes may be recovered and reused for treating one or more additional pre-treated waste textile materials (not shown in Figure 1). Cellulase enzymes may be recovered, for example, by means of dialysis. Advantageously, at the end of the enzymatic treatments, the enzymes are not removed from the mixture. Materials such as, for example, non-cellulosic fibers (e.g., polyester fibers and/or elastane fibers), as well as non-soluble dyes (such as indigo dye and sulphur dye, if present) may be recovered from the solid phase (block 8 of figure 1). The polyester fibers, or other synthetic fibers thus recovered, may be recycled as starting material for preparing filaments and fibers for recycled yarns and fabrics (block 8a).

The obtained liquid mixture including glucose and enzymes is, then, used for the preparation of a microbial culture (block 12 of figure 1) for the production of biocellulose (i.e., bacterial cellulose). To this aim, the mixture is thermally treated to inactivate the enzymes that are present in it. Preferably, the mixture including glucose is sterilized, e.g., by heating, for example, at 121°C in a reactor suitable for the production of microbial cellulose. Optionally, additives may be added to the mixture including glucose (block 10 of figure 1) and sterilized. Optionally, the concentration of glucose in the mixture can be adjusted.

Microbial cellulose producing microorganisms are then added to the liquid mixture including glucose as a starter culture (block 11 of figure 1). The degraded enzymes are no longer active and provide instead a source of Nitrogen and Carbon as nutrients for the microorganisms, e.g. in the form of aminoacids or of parts of aminoacids. Optionally, the concentration of amino acids in the mixture can be adjusted, if necessary. For example, the concentration of amino acids in the mixture can be adjusted by adding peptone to the mixture.

The culture comprising microbial cellulose-producing microorganisms so obtained is then incubated to grow microbial cellulose (block 13 of figure 1). At the end of the incubation, microbial cellulose, usually as a layer of microbial cellulose, is obtained.

The microbial cellulose, e.g., the microbial cellulose layers, are recovered from the one or more containers, washed and, optionally, dried (block 14 of figure 1). The washing may be carried out using an aqueous solution including a bleaching agent. The washed microbial cellulose is then optionally dried. In embodiments, the microbial cellulose, preferably after washing, may be partially dried. For example, partial drying of the microbial cellulose results in a microbial cellulose having a water content in the range from 99% to 80% by weight, preferably from 90% to 80%, more preferably 85%.

The microbial cellulose obtained in block 14 may be advantageously used as the starting cellulosic material in a process for preparing regenerated cellulose fibers. A suitable process for the step of block 15 of fig. 1, is a process used for viscose production. The regenerated cellulose fibers may then be used to manufacture yarns and fabric in a fully circular cycle (block 15a).

The invention will now be further illustrated by reference to the following non limiting examples.

*EXAMPLE 1 - production of bacterial cellulose starting from waste textile material*

*The process of the invention was carried out to produce bacterial cellulose starting from waste textile material.*

*Waste textile material*

An undyed blended waste fabric (fabric composition: 76% viscose, 23% polyester, 1% elastane) was used as waste textile material.

*Pre-treatment with aqueous solution comprising NaOH and urea*

7% NaOH (w/v) and 12% urea (w/v) were dissolved in 40L distilled water and cooled in a climate-controlled room until 0°C, to obtain an aqueous solution of NaOH and urea. A 2 kg fabric was soaked in the cold aqueous solution of NaOH and urea and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 120°C in stenter machine.

*Treatment with cellulase enzymes*

The pre-treated fabric was shredded into small pieces (about 1 cm $\times$ 1 cm), soaked into 40L of citric acid buffer solution (0.05M, pH 5.0), and heated at 50°C in a bioreactor. 40L of Citric Acid Buffer solution (0.05M, pH 5.0) were prepared as follows: 420 gr citric acid monohydrate were measured and dissolved in 35L distilled water. The mixture was agitated until the citric acid monohydrate was completely dissolved. The pH was adjusted to 5.0 using NaOH. Distilled water was added until 40L. 114.28 mL Novozyme Cellic® CTec3 HS cellulase (corresponding to 20 FPU enzyme per gram of cellulose) were added to the solution. After that the enzyme was added to the solution, solution was agitated for 1 min at 100 rpm to homogenously dissolve enzyme. The fabric was, then, incubated in the solution containing cellulase enzymes for 24 hours at 50°C with no agitation. At the end of a treatment, a mixture including a solid phase (mainly non cellulosic materials such as, but limited to, polyester and elastane, and partially degraded cellulosic materials, e.g., partially degraded viscose) and a liquid phase including glucose is obtained.

*Separation of liquid phase from solid phase*

After the incubation, the solid phase of the mixture was separated from the liquid phase of the mixture by filtration via a 0.3 mm sieve. The filtered liquid phase was centrifuged at 9000 RPM for 30 minutes. After centrifuge, the supernatant was taken as a mixture including glucose (e.g., a glucose-rich syrup).

*Preparation of the microbial culture*

Optionally, the mixture including glucose may be concentrated, for example by rotary evaporator. For example, 40L of mixture can be concentrated to a final volume of 10L. The yield of enzymatic reaction was determined using the mixture including glucose, without carry out the concentrating step.

In the present example, according to DNS method, 38.8 gr/L glucose (measured on the 40L mixture including glucose above mentioned) was released from 2 kg blended fabric containing 1.52 kg viscose.

If the mixture is concentrated to a final volume of 10L, the concentration of glucose in the mixture increases up to 155.2 gr/L.

In the present example, according to NREL method, the yield was 91.9% and was calculated as follows:

$$Cellulose\ Conversion\ (\%) = \frac{Glucose\ released\ from\ enzymatic\ hydrolysis\ (g/L) \times Reaction\ volume\ (L)}{Substrate\ weight\ (g) \times Cellulose\ content\ (\%)} \times 0.9 \times 100\%$$

$$Cellulose\ Conversion\ (\%) = \frac{38.8\ (g/L)\ \times\ 40\ (L)}{2000\ (g)\ \times \dfrac{76}{100}} \times 0.9 \times 100\% = 91.9\%$$

where 0.9 is a correction factor for water molecules added upon hydrolysis of the cellulose.

40L of mixture including glucose (38.8 gr/L) and 37. 6L distilled water were mixed to obtain a diluted mixture including glucose having 20 g/L glucose concentration. 77.6L diluted mixture including glucose was autoclaved at 121 °C for 15 min.

The obtained mixture including glucose can be also known as "waste textile glucose " (WTG) medium.

The obtained mixture including glucose can have the following composition:

20.0 gr/L glucose deriving from enzymatic conversion of the cellulose in the waste textile material to glucose;
2.85 ml/L peptone from degraded cellulase enzymes
10.5 gr/L citric acid from the citric acid buffer solution
Water deriving from the mixture obtained after the step of treating with cellulase enzymes.

*Preparation of the starter culture of bacterial cellulose producing bacteria*

A starter bacterial culture was produced as follows. 8L of Hestrin & Schramm (HS) medium were prepared by mixing and autoclaving the following ingredients:

160 gr glucose
40 gr peptone from soybean, enzymatic digest
40 gr yeast extract
27.2 gr di-Sodium hydrogen phosphate dihydrate
12 gr citric acid monohydrate
8L water

0.8L autoclaved HS medium were inoculated with Gluconacetobacter hanseii ATCC 53582 bacteria and incubated at 28°C for 2 days.
Freshly prepared 0.8L bacterial culture were mixed with 7.2Z autoclaved HS medium.
Then, the mixture was stirred at 150 rpm using shaking incubator for 2 days. 8L freshly prepared starter bacterial culture were obtained and ready to use.

*Mixing of the mixture including glucose (WTG medium) with the starter bacterial culture*

77.6L diluted mixture including glucose (20 gr glucoselL) (i.e., the waste textile glucose medium) and 8L freshly prepared starter bacterial culture were mixed aseptically. Then, 4L of the so obtained new bacterial mix was distributed to 21 plastic containers (730 mm width $\times$ 460 mm length $\times$ 193 mm height) and covered with lid. Incubation of the bacterial culture
Covered plastic containers were incubated at 28°Cfor 14 days in a climate-controlled room, to obtain bacterial cellulose.

*Cleaning and drying of the bacterial cellulose*

On average 17 ± 0.35 kg wet and dirty bacterial cellulose layers can be collected from 85.6L bacterial culture. These bacterial cellulose layers were taken out of containers and collected in a reactor. Then, the bacterial cellulose layers were washed with 100L hot water (80°C) having 1% bleach, wherein the bleach contained 5% w/v sodium hypochlorite (i.e., 100L hot water including 0.05% w/v sodium hypochlorite were used) for 3 hours under stirring at 100 rpm. Later, 100L polluted water were discharged and another 100L clean hot water (80°C) were added and solution is stirred at 100 rpm for 3 hours.
On average, 16± 0.32 kg wet and clean bacterial cellulose layers can be obtained from 17± 0.35 kg wet and dirty bacterial cellulose layers.
After these bacterial cellulose layers are dried at 28°C for 12 hours, dry bacterial cellulose is obtained. On average 470 ±10 gr dry bacterial cellulose can be obtained from 16± 0.32 kg wet and clean bacterial cellulose.

**EXAMPLE 2** - *exemplary Room Temperature chemical pre-treatment and enzymatic treatment of an undyed blended waste fabric*

An undyed blended waste fabric (fabric composition: 76% viscose, 23% polyester, 1% elastane) was used as waste textile material.
7% NaOH (w/v) and 12% urea (w/v) were dissolved in 1L distilled water.
A 50 g fabric was soaked in the aqueous solution of NaOH and urea (1L) and pre-treated for 2 hours at room temperature (between 20 and 25°C). After 2 hours, the fabric was neutralized with tap water at 20°C until pH 7. As an alternative, the fabric may be neutralized with weak acid (e.g., acetic acid) washing. Then, the pre-treated fabric was dried at 80°C in an oven.
The pre-treated fabric was shredded into small pieces (e.g., 1 cm $\times$ 1 cm), soaked into 1L citric acid buffer solution (0.05M, pH 5.0), and heated at 50°C in a bioreactor. 2.85 mL Novozyme Cellic CTec3 HS cellulase (corresponding to 20 FPU enzyme per gram of cellulose) were added to the solution. After that, the enzyme was added to the solution,

solution was agitated for 1 minute at 100 rpm to homogeneously dissolve enzyme. The fabric was incubated in the solution containing cellulase enzymes for 24 hours at 50°C with no agitation. At the end of a treatment, a mixture including a solid phase (mainly non cellulosic materials such as polyester and elastane, and partially degraded cellulosic materials, e.g., partially degraded viscose) and a liquid phase including glucose is obtained.

After the incubation, the solid phase of the mixture was separated from the liquid phase of the mixture by filtration via a 0.3 mm sieve. The filtered liquid phase was centrifuged at 9000 RPM for 30 minutes. After centrifuge, the supernatant was taken as the mixture including glucose (e.g., a glucose-rich syrup).

The yield of enzymatic reaction was determined.

According to DNS method, 36.2 gr/L glucose was released from 50 g blended fabric containing 38 g of viscose.

According to NREL method, the yield was 85. 7% and was calculated as follows:

$$Cellulose\ Conversion\ (\%) = \frac{36.2\ (g/L) \times 1\ (L)}{50\ (g) \times \frac{76}{100}} \times 0.9\ \times 100\% = 85.7\%$$

where 0.9 is a correction factor for water molecules added upon hydrolysis of the cellulose.

The mixture including glucose (36.2 gr/L) can be mixed with distilled water to obtain a diluted mixture including glucose having 20 g/L glucose concentration, i. e., to obtain a waste textile glucose (WTG) medium.

### EXAMPLE 3 - *exemplary chemical pre-treatment and enzymatic treatment of an undyed 100% cotton waste fabric*

An undyed waste fabric (fabric composition: 100% cotton) was used as waste textile material.

7% NaOH (w/v) and 12% urea (w/v) were dissolved in 1L distilled water and cooled in a refrigerator until 0°C, to obtain an aqueous solution of NaOH and urea. A 50 g fabric was soaked in the aqueous solution of NaOH and urea (1L) and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 80°C in an oven.

The pre-treated fabric was shredded into small pieces (e.g., 1 cm × 1 cm), soaked into 1L citric acid buffer solution (0.05M, pH 5.0), and heated at 50°C in a bioreactor. 3.76 mL Novozyme Cellic® CTec3 HS cellulase (corresponding to 20 FPU enzyme per gram of cellulose) were added to the solution. After that the enzyme was added to the solution, solution was agitated for 1 min at 100 rpm to homogenously dissolve enzyme. The fabric was, then, incubated in the solution containing cellulase enzymes for 96 hours at 50°C with no agitation. At the end of a treatment, a mixture including a solid phase (mainly partially degraded cellulosic materials, e.g., partially degraded cotton) and a liquid phase including glucose is obtained.

The mixture including a solid phase and a liquid phase was centrifuged at 9000 RPM for 30 minutes. After centrifuge, the supernatant was taken as the mixture including glucose (e.g., a glucose-rich syrup).

The yield of enzymatic reaction was determined.

According to DNS method, 51.3 gr/L glucose is released from 50 g 100% cotton fabric.

According to NREL method, the yield was 92.34%and was calculated as follows:

$$Cellulose\ Conversion\ (\%) = \frac{51.3\ (g/L) \times 1\ (L)}{50\ (g) \times \frac{100}{100}} \times 0.9\ \times 100\% = 92.3\%$$

where 0.9 is a correction factor for water molecules added upon hydrolysis of the cellulose.

The mixture including glucose (51.3 gr/L) can be mixed with distilled water to obtain a diluted mixture including glucose having 20 g/L glucose concentration, i.e., to obtain a waste textile glucose (WTG) medium.

### EXAMPLE 4 - *exemplary chemical pre-treatment and enzymatic treatment of an undyed blended waste fabric*

An undyed blended waste fabric (fabric composition: 76% viscose, 23% polyester, 1% elastane) was used as waste textile material.

7% NaOH (w/v) and 12% urea (w/v) were dissolved in 1L distilled water and cooled in a refrigerator until 0°C, to obtain an aqueous solution of NaOH and urea. A 50 g fabric was soaked in the cold aqueous solution of NaOH and urea (1L) and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 80°C in an oven.

The pre-treated fabric was shredded into small pieces (e.g., 1 cm × 1 cm), soaked into 1L citric acid buffer solution (0.05M, pH 5.0), and heated at 50°C in a bioreactor. 2.85 mL Novozyme Cellic® CTec3 HS cellulase (corresponding to 20 FPU enzyme per gram of cellulose) were added to the solution. After that the enzyme was added to the solution, solution was agitated for 1 min at 100 rpm to homogenously dissolve enzyme. The fabric was, then, incubated in the solution containing cellulase enzymes for 24 hours at 50°C with no agitation. At the end of a treatment, a mixture including a solid phase (mainly non cellulosic materials such as polyester and elastane, and partially degraded cellulosic materials, e.g., partially degraded viscose) and a liquid phase including glucose is obtained.

After the incubation, the solid phase of the mixture was separated from the liquid phase of the mixture by filtration via a 0.3 mm sieve. The filtered liquid phase was centrifuged at 9000 RPM for 30 minutes. After centrifuge, the supernatant was taken as the mixture including glucose (e.g., a glucose-rich syrup).

The yield of enzymatic reaction was determined.

According to DNS method, 38.4 gr/L glucose was released from 50 g blended fabric containing 38 g of viscose.

According to NREL method, the yield was 90.9% and was calculated as follows:

$$Cellulose\ Conversion\ (\%) = \frac{38.4\,(g/L) \times 1\,(L)}{50\,(g) \times \frac{76}{100}} \times 0.9 \times 100\% = 90.9\%$$

where 0.9 is a correction factor for water molecules added upon hydrolysis of the cellulose.

The mixture including glucose (38.4 gr/L) can be mixed with distilled water to obtain a diluted mixture including glucose having 20 g/L glucose concentration, i.e., to obtain a waste textile glucose (WTG) medium.

***EXAMPLE 5** - exemplary chemical pre-treatment and enzymatic treatment of an indigo dyed blended waste fabric*

An indigo dyed blended waste fabric (fabric composition: 99% cotton, 1% elastane; indigo dyed) was used as waste textile material.

7% NaOH (w/v) and 12% urea (w/v) were dissolved in 7.2Z distilled water and cooled in a refrigerator until 0°C, to obtain an aqueous solution of NaOH and urea. A 60 g fabric piece was soaked in the cold aqueous solution of NaOH and urea (1.2L) and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 80°C in an oven. The pre-treated fabric was shredded into small pieces (e.g., 1 cm × 1 cm), soaked into 1.2L citric acid buffer solution (0.05M, pH 5.0), and heated at 50°C in a bioreactor. 4.47 mL Novozyme Cellic® CTec3 HS cellulase (corresponding to 20 FPU enzyme per gram of cellulose) were added to the solution. After that the enzyme was added to the solution, solution was agitated for 1 min at 100 rpm to homogenously dissolve enzyme. The fabric was, then, incubated in the solution containing cellulase enzymes for 24 hours at 50°C with no agitation. At the end of a treatment, a mixture including a solid phase (mainly partially degraded cellulosic materials such as cotton and non-cellulosic elastane) and a liquid phase including glucose is obtained.

After the incubation, the solid phase of the mixture was separated from the liquid phase of the mixture by filtration via a 0.3 mm sieve. The filtered liquid phase was centrifuged at 9000 RPM for 30 minutes. After centrifuge, the supernatant was taken as the mixture including glucose (e.g., a glucose-rich syrup) and the precipitants, including indigo dye, were collected.

The mixture including glucose (i.e., the waste textile glucose solution, i.e., the glucose rich syrup), can be mixed with distilled water to obtain a diluted mixture including glucose having 20 g/L glucose concentration, i.e., to obtain a waste textile glucose (WTG) medium.

***EXAMPLE 6** - exemplary chemical pre-treatment and enzymatic treatment of a sulphur dye dyed blended waste fabric*

A sulphur dye dyed blended waste fabric (fabric composition: 92.5% cotton, 5% polyester, 2.5% elastane; sulphur dye) was used as waste textile material.

7% NaOH (w/v) and 10.5% urea (w/v) were dissolved in 2L distilled water and cooled in a refrigerator until 0°C, to obtain an aqueous solution of NaOH and urea. A 100 g fabric was soaked in the cold aqueous solution of NaOH and urea (2L) and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 80°C in an oven.

The pre-treated fabric was shredded into small pieces (e.g., 1 cm × 1 cm), soaked into 2L citric acid buffer solution (0.05M, pH 5.0), and heated at 50°C in a bioreactor. 6.95 mL Novozyme Cellic® CTec3 HS cellulase (corresponding to 20 FPU enzyme per gram of cellulose) were added to the solution. After that the enzyme was added to the solution, solution was agitated for 1 min at 100 rpm to homogenously dissolve enzyme. The fabric was, then, incubated in the solution containing cellulase enzymes for 24 hours at 50°C with no agitation. At the end of a treatment, a mixture including a solid phase (mainly non-cellulosic materials including, but not limited to, polyester and elastane and partially degraded cellulosic materials such as cotton) and a liquid phase including glucose is obtained.

After the incubation, the solid phase of the mixture was separated from the liquid phase of the mixture by filtration via a 0.3 mm sieve. The filtered liquid phase was centrifuged at 9000 RPM for 30 minutes. After centrifuge, the supernatant was taken as the mixture including glucose (e.g., a glucose-rich syrup) and the precipitants, including sulphur dye, are collected.

The mixture including glucose, can be mixed with distilled water to obtain a diluted mixture including glucose having 20 g/L glucose concentration, i.e., to obtain a waste textile glucose (WTG) medium.

### EXAMPLE 7 - exemplary chemical pre-treatment and enzymatic treatment of a black reactive dyed blended waste fabric

A black reactive dye dyed blended waste fabric (fabric composition: 90% cotton, 6% polyester, 4% elastane; black reactive dye) was used as waste textile material.

7% NaOH (w/v) and 14.0% urea (w/v) were dissolved in 2L distilled water and cooled in a refrigerator until 0°C, to obtain an aqueous solution of NaOH and urea. A 100 g fabric was soaked in the cold aqueous solution of NaOH and urea (2L) and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 80°C in an oven.

The pre-treated fabric was shredded into small pieces (e.g., 1 cm × 1 cm), soaked into 2L citric acid buffer solution (0.05M, pH 5.0), and heated at 50°C in a bioreactor. 6.77 mL Novozyme Cellic® CTec3 HS cellulase (corresponding to 20 FPU enzyme per gram of cellulose) were added to the solution. After that the enzyme was added to the solution, solution was agitated for 1 min at 100 rpm to homogenously dissolve enzyme. The fabric was, then, incubated in the solution containing cellulase enzymes for 24 hours at 50°C with no agitation. At the end of a treatment, a mixture including a solid phase (mainly non-cellulosic materials including but not limited to polyester and elastane and partially degraded cellulosic materials such as cotton) and a liquid phase including glucose is obtained.

After the incubation, the solid phase of the mixture was separated from the liquid phase of the mixture by filtration via a 0.3 mm sieve. The filtered liquid phase was centrifuged at 9000 RPM for 30 minutes. After centrifuge, the supernatant was taken as the mixture including glucose (e.g., a glucose-rich syrup), further including the black reactive dye. The dye was removed from the liquid mixture through activated carbon column chromatography. Particularly, to remove the dye, the glucose-rich syrup was poured into activated carbon column with 2 mL/minflow rate. A 5 cm × 50 cm column contains 1 kg activated carbon beads with a diameter of 2.88 mm ± 0.26 mm.

The mixture obtained after the removal of the dye can be mixed with distilled water to obtain a diluted mixture including glucose having 20 g/L glucose concentration, i.e., to obtain a waste textile glucose (WTG) medium.

### EXAMPLE 8 - Comparison of Hestrin Schramm (HS) and Waste Textile Glucose (WTG) media

The bacterial strain Gluconacetobacter hansenii ATCC 53582 was grown in 500 mL of Waste Textile Glucose (WTG) Medium of Hestrin and Schramm (HS) Medium, at an incubation temperature of 28°C - 30°C, for 14 days, in a close-lid plastic box. At the end of the incubation, the wet bacterial cellulose obtained was squeezed or pressed with mechanical devices to remove excess of culture medium. The amount of the wet bacterial cellulose obtained was, then, determined.

| Table 1 - Wet Bacterial Cellulose Amount (gr) ||
| Waste Textile Glucose (WTG) Medium | Hestrin and Schramm (HS) Medium |
| --- | --- |
| 124.6 | 101.3 |
| 111.4 | 87.3 |
| 116.1 | 94.3 |

It was observed that the use of waste textile glucose medium increases wet bacterial cellulose yield around 20%, with respect to the HS medium.

**EXAMPLE 9 - Comparison of additives (TiBALDH, Glycerol, Ascorbic Acid)**

The bacterial strain Gluconacetobacter hansenii ATCC 53582 was grown in 500 mL of waste textile glucose medium at an incubation temperature of 28°C - 30°C, for 14 days, in a close-lid plastic box.

Different experiments were carried out to test the effect of the addition of different additives (TiBALDH, Glycerol, Ascorbic Acid) in different concentration on the production of bacterial cellulose.

At the end of the incubation, the amount of the wet bacterial cellulose obtained was determined. Comparisons of wet bacterial amount change (%) in Table 2, 3, 4, and 5 are based on data in Table 1.

_TiBALDH_

The first additive tested was Titanium (IV) bis-(ammonium lactato)dihydroxide solution (TiBALDH), CAS Number 65104-06-5.

| Table 2 | |
| --- | --- |
| TiBALDH Concentration (%) | Wet BC amount change (%) |
| 0.0050 | 7.04 |
| 0.0250 | 18.10 |
| 0.0500 | 24.13 |
| 0.2500 | 55.30 |
| 0.5000 | 17.10 |
| 1.0000 | -26.79 |

According to the obtained results, 0.25% TiBALDH addition to the waste textile glucose medium causes the highest (+SS. 30%) increase in wet BC amount obtained.

_Glycerol_

The second additive tested was glycerol, CAS Number: 56-81-5.

| Table 3 | |
| --- | --- |
| Glycerol Concentration (%) | Wet BC amount change (%) |
| 0.5 | 10.93 |
| 1.0 | 19.74 |
| 2.0 | 10.15 |

According to the obtained results, 1% glycerol addition to the waste textile glucose medium causes the highest (+ 19. 74%) increase in wet BC amount.

_Ascorbic Acid (Vitamin C)_

The third additive tested was ascorbic acid (Vitamin C), CAS Number: 50-81-7.

| Table 4 | |
| --- | --- |
| Vitamin C Concentration (%) | Wet BC amount change (%) |
| 0.25 | 6.75 |
| 0.50 | 9.25 |
| 1.00 | 8.13 |

(continued)

| Table 4 | |
|---|---|
| Vitamin C Concentration (%) | Wet BC amount change (%) |
| 2.00 | 6.95 |
| 3.00 | 5.57 |
| 4.00 | 2.96 |

According to the obtained results, 0.5% ascorbic acid addition to the waste textile glucose medium causes the highest (+9.25%) increase in wet BC amount.

Combinations of additives

Different combinations of the three additives TiBALDH, Glycerol and Ascorbic Acid were tested.

| Table 5 | | | |
|---|---|---|---|
| 0.5% Vitamin C | 1% Glycerol | 0.25% TiBALDH | Wet BC amount change (%) |
| + | - | - | 9.25 |
| - | + | - | 19.74 |
| - | - | + | 55.30 |
| + | + | - | 13.56 |
| + | - | + | 28.30 |
| - | + | + | 58.04 |
| + | + | + | 16.36 |

According to the obtained results, the highest wet BC amount is achieved by addition of %1 glycerol and %0.25 TiBALDH (58.04%).

EXAMPLE 10 - Comparison of waste textile material before and after the pre-treatment with an aqueous solution comprising NaOH and urea

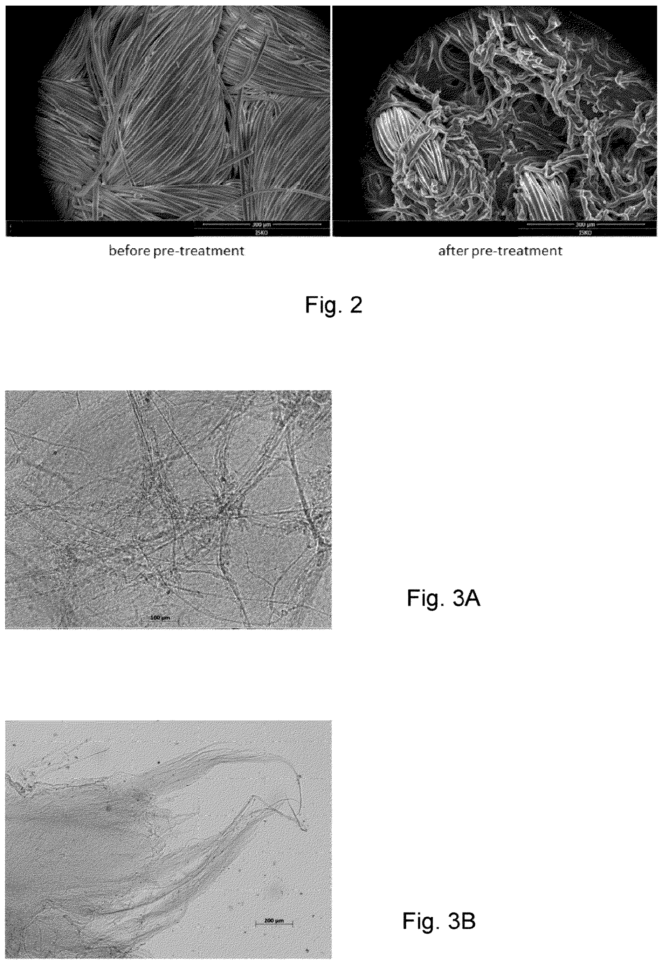

A blended wastefabric (fabric composition: 76% viscose, 23% polyester, 1% elastane) was treated with a solution of 7% NaOH (w/v) and 12% urea (w/v) for 5 hours at 0°C. Before and after the treatment, images of the fabric were taken and analyzed using a scanning electron microscope (SEM). As can be observed from Figure 2, the fabric after the treatment is swelled, i.e., the fibers in the fabric are separated with respect to the fibers in the fabric before the treatment.

EXAMPLE 11 - Effects of pre-treatment with an aqueous solution comprising NaOH and urea at room temperature and at 50°C on polyester

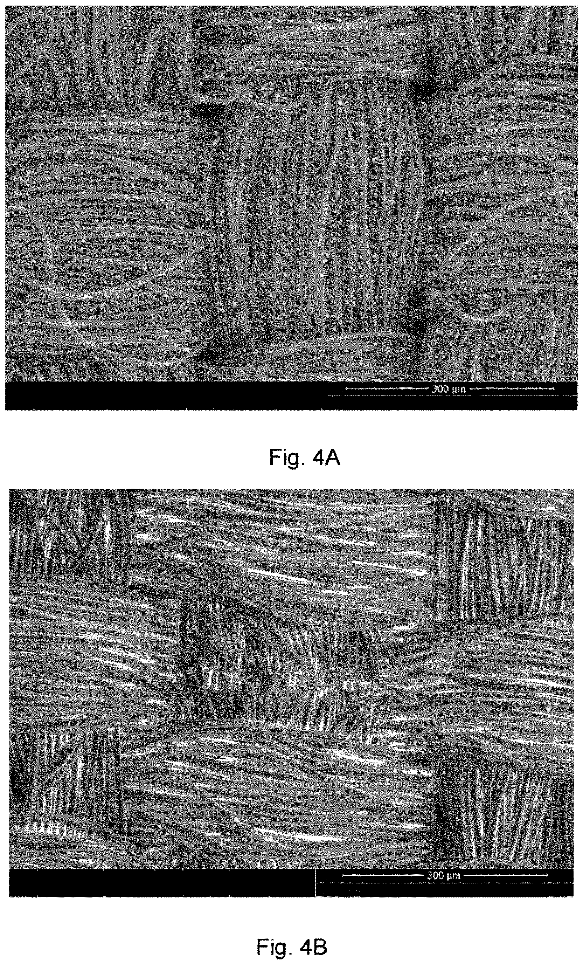

Two samples of a 100% raw polyester fabric were treated with a solution of 7% NaOH (w/v) and 12% urea (w/v) for 5 hours at room temperature and at 50°C, respectively. After the treatments, images of the samples were taken and analyzed using a scanning electron microscope (SEM). As can be observed from Figure 4A, the pre-treatment carried out at room temperature does not damage the polyester fibers. Conversely, pre-treatment carried out at 50°C damage the polyester fibers (Figure 4B).

EXAMPLE 12 - Comparison of waste textile material before and after the pre-treatment with an aqueous solution comprising NaOH and urea (0°C and room temperature) and enzymatic treatment

Samples of a blended waste fabric (fabric composition: 76% viscose, 23% polyester, 1% elastane) were pre-treated with a solution of 7% NaOH (w/v) and 12% urea (w/v) for 5 hours at 0°C or at room temperature and, subsequently,

*treated with cellulase enzymes. Before and after the treatments, images of the samples were taken and analyzed using a scanning electron microscope (SEM). Figure 5A shows a SEMimage of the sample before the pre-treatment and an enzymatic treatment. Figure 5B shows the sample material after the pre-treatment carried out at 0°C and the treatment with cellulase. Figure 5C shows the sample material after the pre-treatment carried out at room temperature and the treatment with cellulase. As can be observed the process of the invention does not damage the polyester fibers.*

### EXAMPLE 13 - *characterization of glucose obtained after the treatment with cellulase*

*A blended waste fabric (fabric composition: 76% viscose, 23% polyester, 1% elastane) and a 100% cotton waste fabric were pre-treated with a solution of NaOH and urea, and then treated with cellulase enzymes, according to the process of the invention. The glucose obtained after the step of treatment with cellulase enzymes was analyzed by Nuclear Magnetic Resonance (NMR). The following result were obtained.*

*Blended fabric: $^1$H-NMR of glucose in $D_2O$; 40.7% $\alpha$-glucose, 59.3% $\beta$-glucose 5.21 ppm (bs, $H_{1-\alpha}$), 4.63 ppm (d, J=7.6 Hz, $H_{1-\beta}$), 3.87 ppm (d, J=12.0 Hz, $H_{6-\alpha}$), 3.81 ppm (d, J=10.9 Hz, $H_{5-\alpha}$ & $H_{6-\alpha}$), 3. 77-3. 64ppm (m, $H_{6-\alpha}$ & $H_{3-\alpha}$ & $H_{6-\beta}$), 3.52ppm (d, J=8.0 Hz, $H_{2-\alpha}$), 3.46 ppm (dd, J=18.2, 9.1 Hz, $H_{3-\beta}$ & $H_{5-\beta}$), 3.38 ppm (dd, J=15.3, 8.6 Hz, $H_{4-\alpha}$ & $H_{4-\beta}$), 3.23 ppm (t, J=8.1 Hz, $H_{2-\beta}$)*

*100% Cotton fabric: $^1$H-NMR of glucose in $D_2O$; 43.3% $\alpha$-glucose, 56. 7% $\beta$-glucose 5.23 ppm (d, J=3.7 Hz, $H_{1-\alpha}$), 4.65 ppm (d, J=7.9 Hz, $H_{1-\beta}$), 3.90 ppm (dd, J=12.3, 1.9 Hz, $H_{6-\beta}$), 3.87-3.81 ppm (m, $H_{5-\alpha}$ or $H_{6-\alpha}$), 3.76 ppm (dd, J=12.5, 5.5 Hz, $H_{6-\alpha}$), 3.75-3.69 ppm (m, $H_{3-\alpha}$ & $H_{6-\beta}$), 3.53 ppm (dd, J=9.8, 3.7 Hz, $H_{2-\alpha}$), 3.50-3.45 ppm (m, $H_{3-\beta}$ & $H_{5-\beta}$), 3.41 ppm (dt, J=9.4, 6.1 Hz, $H_{4-\alpha}$ & $H_{4-\beta}$), 3.24 ppm (dd, J=9.1, 8.1 Hz, $H_{2-\beta}$)*

### EXAMPLE 14 - *structure of an exemplary bacterial cellulose obtained according to the process of the invention*

*A sample of an exemplary bacterial cellulose obtained according to the process of the invention was analyzed and characterized.*

*The following results were obtained.*

*Viscosity: 980 ml/g (measured according to ISO 5351:2010)*
*Molecular weight distribution: Mn=88047g/mol, Mw= 510577 g/mol DI=5.8 (SEC analysis in 0.5% (w/v) LiCl/DMAc mobile phase)*
*% Cellulose in the bacterial cellulose >99%*
*% Hemicellulose in the bacteria cellulose <1%; Arabinose 0. 08 g/kg, Galactose 0.03 g/kg, Xylose 0.05 g/kg, Mannose 0.4 g/kg, Glucose 882 g/kg (SCAN-CM 71:09)*
*% Ash content <0.5% (According to ISO 1762:2019)*

*Figure 3A and Figure 3B show the structure of the bacterial cellulose, as observed by Light Microscopy, in two magnifications, namely 150X in fig. 3A and 75X in fig. 3B. As can be observed from Figure 3A and Figure 3B, the structure of the bacterial cellulose is striated with a network of both thin and thicker fibers.*

### Example 15 - *washing of bacterial cellulose*

*After the fermentation of bacterial cellulose (BC) is complete, BC layers are taken out of fermentation container and are placed into laundry bags that are closed with resealable Velcro. The laundry bags are made of a fabric having mesh size between 50 micrometers and 100 micrometers, preferably 75 micrometers. Laundry bags are squeezed with a cloth wringer to remove excess bacterial solution. After that, laundry bags are located into an industrial washing machine for inactivating and removing bacteria and whitening BC layers. Washing protocol includes the following:*

*Liquor ratio by weight of bacterial cellulose layers/water: 1/10, preferably 1/4*
*Whitening agent: 1% bleach*
*Temperature: 30°C*
*Duration: 30 min*

### Example 16 - *Exemplary room temperature chemical pre-treatment and enzymatic treatment of an undyed 100% cotton waste fabric*

*An undyed waste fabric (fabric composition: 100% cotton) was used as waste textile material.*
*7% NaOH (w/v) and 12% urea (w/v) were dissolved in 1L distilled water.*
*A 50 g fabric was soaked in the aqueous solution of NaOH and urea (1L) and pretreated for 2 hours at room*

temperature (between 20 and 25°C). After 2 hours, the fabric was neutralized with tap water at 20°C until pH 7. As an alternative, the fabric may be neutralized with weak acid (e.g., acetic acid) washing. Then, the pre-treated fabric was dried at 80°C in an oven.

The pre-treated fabric was shredded into small pieces (e.g., 1 cm × 1 cm), soaked into 1L citric acid buffer solution (0.05M, pH 5.0), and heated at 50°C in a bioreactor. 3.76 mL Novozyme Cellic CTec3 HS cellulase (corresponding to 20 FPU enzyme per gram of cellulose) were added to the solution. After that, the enzyme was added to the solution, solution was agitated for 1 minute at 100 rpm to homogeneously dissolve enzyme. The fabric was incubated in the solution containing cellulase enzymes for 24 hours at 50°C with no agitation. At the end of a treatment, a mixture including a solid phase (partially degraded cellulosic materials, e.g., partially degraded cotton) and a liquid phase including glucose is obtained.

After the incubation, the solid phase of the mixture was separated from the liquid phase of the mixture by filtration via a 0.3 mm sieve. The filtered liquid phase was centrifuged at 9000 RPM for 30 minutes. After centrifuge, the supernatant was taken as the mixture including glucose (e.g., a glucose-rich syrup). The yield of enzymatic reaction was determined.

According to DNS method, 45.62 gr/L glucose was released from 50 gr 100% cotton fabric. According to NREL method, the yield was 82.12% and was calculated as follows:

$$Cellulose\ Conversion\ (\%) = \frac{45.62\ (g/L) \times 1\ (L)}{50\ (g) \times \frac{100}{100}} \times 0.9 \times 100\% = 82.12\%$$

where 0.9 is a correction factor for water molecules added upon hydrolysis of the cellulose.

The mixture including glucose (45.62 gr/L) can be mixed with distilled water to obtain a diluted mixture including glucose having 20 g/L glucose concentration, i.e., to obtain a waste textile glucose (WTG) medium.

### Example 17 - Enzymatic activity comparison at different chemical pretreatment temperatures: -20C, 0C, and room temperature

An undyed waste textile fabric (fabric composition: 76% viscose, 23% polyester, 1% elastane) was used (as in Example 1 and Example 2).

NaOH and urea pretreatment was carried out at -20°C, for 24 hrs. Then, same process of Example 1 was carried out. The cellulose conversion yield obtained from enzymatic treatment was 89.6%.

According to Example 1, NaOH and urea pretreatment was carried out at 0°C, for 5 hrs. The cellulose conversion yield obtained from enzymatic treatment was 91.9% (see also Example 1).

According to Example 2, NaOH and urea pretreatment was carried out at room temperature (between 20 and 25°C), for 2 hrs. The cellulose conversion yield obtained from enzymatic treatment was 87.5% (see also Example 2).

Result: carrying out the pre-treating step with NaOH and urea at room temperature allows to obtain high conversion of cellulose to glucose while greatly reducing the duration of the pre-treatment.

### Example 18 - exemplary chemical pre-treatment and enzymatic treatment of an undyed 100% cotton waste fabric

An undyed waste textile fabric (fabric composition: 100% cotton) was used as waste textile material.

The same process of Example 3 was carried out, using the enzyme AB Enzyme-Flashzyme Plus 200 instead of Novozymes Cellic® CTec3 HS.

| Table 6 | | | | | | |
|---|---|---|---|---|---|---|
| | 1 day | 2 days | 3 days | 4 days | 5 days | 6 days |
| | 24 hours | 48 hours | 72 hours | 96 hours | 120 hours | 144 hours |
| Cellulose to glucose conversion rate (% yield) | 3.68 | 31.14 | 48.42 | 57.62 | 69.69 | 93.51 |

The cellulose conversion yield obtained from enzymatic treatment was 93.51%, after 144 hours.

*Example 19 - recycling of the cellulase enzymes*

*An undyed waste textile fabric (fabric composition: 100% cotton) was used as waste textile material.*

*7% NaOH (w/v) and 12% urea (w/v) were dissolved in 1L distilled water and cooled in a refrigerator until 0°C, to obtain an aqueous solution of NaOH and urea. A 50 g fabric was soaked in the aqueous solution of NaOH and urea (1L) and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 80°C in an oven.*

*The pre-treated fabric was shredded into small pieces (e.g., 1 cm × 1 cm), soaked into 1L citric acid buffer solution (0.05M, pH 5.0), and heated at 50°C in a bioreactor. 3.76 mL Novozyme Cellic® CTec3 HS cellulase (corresponding to 20 FPU enzyme per gram of cellulose) were added to the solution. After that the enzyme was added to the solution, solution was agitated for 1 min at 100 rpm to homogenously dissolve enzyme. The fabric was, then, incubated in the solution containing cellulase enzymes for 24 hours at 50°C with no agitation. At the end of a treatment, a mixture including a solid phase (mainly partially degraded cellulosic materials, e.g., partially degraded cotton) and a liquid phase including glucose is obtained.*

*The mixture including a solid phase and a liquid phase was centrifuged at 9000 RPM for 30 minutes. After centrifuge, the supernatant was taken as the mixture including glucose (e.g., a glucose-rich syrup).*

*After centrifugation the enzymes were removed from the supernatant (i.e., the mixture including glucose) by means of dialysis (Dialysis cassette: Thermo Scientific - Slide-A-Lyzer cassette (10 KMWCO)), as follows.*

*1 L of 0. 05M citric acid (pH 5) buffer was prepared and placed on ice.*

*The dialysis cassette was placed into the buffer solution and stirring was performed with a magnetic stirrer for 3 hours, to remove glucose resulting from cellulose degradation via enzymatic recycling from the mixture within the dialysis cassette.*

*Dialysis was performed under cold conditions to prevent enzyme degradation. The 10 kDa dialysis cassette was selected based on the approximate molecular weight of cellulase to prevent enzyme passage through the dialysis cassette.*

*After 3 hours, the solution from the dialysis cassette was used to contact the solid phase (mainly partially degraded cellulosic materials, e.g., partially degraded cotton) obtained as above discussed for the continuous enzymatic degradation.*

*After incubation for 24 hours at 50°C with no agitation (total time of enzymatic treatment: 48 hours), the obtained mixture including a solid phase and a liquid phase including glucose was centrifuged; after centrifuge, the enzymes were removed from the supernatant and reused again contact the solid phase obtained after centrifugation.*

*The same steps of enzymatic treatment, separation of the liquid phase from the solid phase, recovering of enzymes by dialysis and reuse of the enzymes were performed two more times (72 hours and 96 hours of enzymatic treatment).*

*Cellulose to glucose conversion (% yield) was 79.77% after the second time usage of enzymatic solution (i.e., after a total of 48 hours of enzyme treatment).*

*Cellulose to glucose conversion (% yield) was 99.18% after the fourth time usage of enzymatic solution (i.e., after a total of 96 hours of enzyme treatment).*

*Example 20 - effect of shredding on the amount of dissolved cellulose*

*An undyed waste textile fabric (fabric composition: 76% viscose, 23% polyester, 1% elastane) was used as waste textile material.*

*A 2 g fabric sample was soaked in the aqueous solution of NaOH and urea and pre-treated. After the pretreatment, the fabric was neutralized with tap water at 20°C until pH 7. Then, the pre-treated fabric was dried at 80°C in an oven. The weight of the pretreated fabric obtained was 1.86 g.*

*A 2 g fabric sample was shredded to small pieces. The shredded material was soaked in the aqueous solution of NaOH and urea and pre-treated. After the pretreatment, the fabric was neutralized with tap water at 20°C until pH 7. Then, the pre-treated fabric was dried at 80°C in an oven. The weight of the pretreated shredded fabric obtained was 1.48 g.*

*As can be observed, when the fabric was not shredded before the treatment, 0.14 of cotton were lost during the pre-treatment, corresponding to a cellulose loss of 7%. Conversely, when the fabric was shredded before the treatment, 0.52 of cotton were lost during the pre-treatment, corresponding to a cellulose loss of 26%.*

*Shredding the waste textile material after the pre-treatment, allows to provide the cellulase enzyme with a higher amount of cellulose which would result, in turn, in a higher amount of glucose at the end of the enzymatic conversion of cellulose to glucose.*

*Example 21* - *exemplary comparison of chemical pre-treatment and non-pretreatment of an undyed blended waste fabric*

*An undyed blended waste fabric (fabric composition: 76% viscose, 23% polyester, 1% elastane) was used as waste textile material.*

*Chemical pre-treatment*

*7% NaOH (w/v) and 12% urea (w/v) were dissolved in 100 mL distilled water and cooled in a refrigerator until 0°C, to obtain an aqueous solution of NaOH and urea. A 5 g fabric was soaked in the cold aqueous solution of NaOH and urea and pre-treated for 5 hours at 0°C. After 5 hours, the fabric was neutralized with tap water at 20°C until pH 7. As an alternative, the fabric may be neutralized with a weak acid (e.g., acetic acid) washing. Then, the pre-treated fabric was dried at 80°C in an oven. The pre-treated fabric was shredded into small pieces (e.g., 1 cm × 1 cm).*

*Non-pretreatment*

*A 5 g fabric was shredded into small pieces (e.g., 1 cm × 1 cm).*

*Treatment with cellulase enzymes*

*Pretreated and non-pretreated fabrics were soaked into 100 mL citric acid buffer solution (0.05M, pH 5. 0), and heated at 50°C in an orbital shaker. 0.28 mL Novozyme Cellic® CTec3 HS cellulase (corresponding to 20 FPU enzyme per gram of cellulose) were added to the solution. After that the enzyme was added to the solutions, solutions were agitated for 1 min at 100 rpm to homogenously dissolve enzyme. The fabrics were, then, incubated in the solutions containing cellulase enzymes for 24 hours at 50°C with no agitation. At the end of a treatment, two mixtures including a solid phase (mainly non cellulosic materials such as polyester and elastane, and partially degraded cellulosic materials, e.g., partially degraded viscose) and a liquid phase including glucose are obtained.*

*After the incubation, the solid phase of the mixtures was separated from the liquid phase of the mixtures by filtration via a 0.3 mm sieve. The filtered liquid phases were centrifuged at 9000 RPM for 30 minutes. After centrifuge, the supernatants were taken as the mixture including glucose (e.g., a glucose-rich syrup).*

*The yield of enzymatic reaction:*

*- Chemical Pretreatment*

*According to DNS method, 38.4 gr/L glucose was released from 5 g blended fabric containing 3.8 g of viscose.*

*According to NREL method, the yield was 90.9% and was calculated as follows:*

$$Cellulose\ Conversion\ (\%) = \frac{37.7\ (g/L) \times 0.1\ (L)}{5\ (g) \times \frac{76}{100}} \times 0.9 \times 100\% = 89.3\%$$

*where 0.9 is a correction factor for water molecules added upon hydrolysis of the cellulose.*

*- Non-pretreatment*

*According to DNS method, 12.7 gr/L glucose was released from 5 g blended fabric containing 3.8 g of viscose.*

*According to NREL method, the yield was 90.9% and was calculated as follows:*

$$Cellulose\ Conversion\ (\%) = \frac{12.7\ (g/L) \times 0.1\ (L)}{5\ (g) \times \frac{76}{100}} \times 0.9 \times 100\% = 30.1\%$$

*where 0.9 is a correction factor for water molecules added upon hydrolysis of the cellulose.*

*As can be observed, pre-treating the waste textile material according to the invention allows to increase the yield of the conversion of cellulose to glucose from 30.1% to 89.3%.*

The above examples provide evidence that the invention process provides a way of recycling cotton and cellulosic fibers and also of recycling polyester or other synthetic fibers from a waste textile, namely from fabrics. Cellulosic fibers, are converted into glucose that is used to produce microbial cellulose that in turn is dissolved and transformed into viscose or similar cellulosic fibers, thus reaching a full recycling cycle of the initial cellulosic fibers.

Also, degradation of polyester fibers in the pre-treatment step with the claimed solution including both NaOH and urea at the claimed temperature range, in particular at room temperature (20°C±5°C) is greatly reduced with respect to prior art treatment, so that the polyester recovered after enzymatic conversion of the cellulosic fibers is suitable to be recycled and spun again into filaments of recycled polyester.

Additionally, the process provides that the enzymes used in the enzymatic conversion of cellulose may consist of cellulase enzymes. It also provides the advantage that the enzyme(s) present in the glucose and containing solution may be thermally inactivated so that said glucose containing solution may be directly used in the following step of culturing cellulose producing microorganisms. Here, the inactivated enzymes act as a source of nutrients for said microorganisms.

The process is particularly advantageous in recycling of blend yarns of synthetic fibers and cellulosic fibers where it would be difficult to recycle the cellulosic fibers as such, e.g. by a mechanical process that maintains the fibers in a fiber form. As an example, the process is useful when the cellulosic fibers are cotton fibers deriving from already recycled fabrics and yarns and thus having an average short length that would result in a length of the recycled fibers too short to be used for a yarn.

**Claims**

1. A process for the production of microbial cellulose from a waste textile material, said waste textile material comprising cellulosic fibers including cellulose, said process comprising the following steps:

    a) providing a waste textile material;
    b) pre-treating said waste textile material with an aqueous solution comprising NaOH and urea, to obtain a pre-treated textile material, wherein said step of pre-treating said waste textile material with an aqueous solution comprising NaOH and urea is carried out at a temperature in the range from -25°C to +30°C;
    c) treating said pre-treated textile material with cellulase, to convert said cellulose into glucose, whereby a mixture including a solid phase and a liquid phase is obtained;
    d) separating said liquid phase from said solid phase, to obtain a liquid mixture including glucose;
    e) preparing a microbial culture using said mixture including glucose, wherein said microbial culture comprises microbial cellulose producing-microorganisms;
    f) incubating said microbial culture to obtain microbial cellulose.

2. A process of enzymatic saccharification of a waste textile material, said waste textile material comprising cellulosic fibers including cellulose, said process comprising the following steps:

    a) providing a waste textile material;
    b) pre-treating said waste textile material with an aqueous solution comprising NaOH and urea, to obtain a pre-treated textile material, wherein said step of pre-treating said waste textile material with an aqueous solution comprising NaOH and urea is carried out at a temperature in the range from -25°C to +30°C;
    c) treating said pre-treated textile material with cellulase, to convert said cellulose into glucose, whereby a mixture including a solid phase and a liquid phase is obtained,

    wherein said pre-treated textile as obtained in step b) is shred into a plurality of pieces having dimension suitable for enzymatic saccharification before step c).

3. The process according to claim 1 or 2, wherein said waste textile material comprises cellulosic fibers and non-cellulosic fibers and is selected from yarns, fabrics and articles containing said fabrics..

4. The process according to any claim from 2 or 3, wherein said cellulosic fibers are selected from cotton fibers, hemp fibers, flax (linen) fibers, jute fibers, viscose fibers, lyocell fibers, modal fibers, acetate fibers, cupro fibers and mixture thereof, preferably said fibers being recycled fibers and wherein said non-cellulosic fibers are selected from polyester fibers, polyamide fibers, elastane fibers, polypropylene (PP) fibers, acrylic fibers, and mixtures thereof, preferably said fibers being recycled fibers.

5. The process according to any previous claim, wherein the amount of NaOH in said aqueous solution comprising NaOH and urea is in the range from 1% to 15% w/v, preferably from 2% to 10% w/v, more preferably from 3% to 9% w/v, the amount of urea in said aqueous solution is in a range from 1% to 25% w/v, preferably from 3% to 20% w/v, more preferably from 5% to 15% w/v.

6. The process according to any previous claim, wherein the ratio by weight of NaOH to urea (NaOH/urea) is in the range of 1.0/1.0 to 1.0/2.0, preferably 1.0/1.5 to 1.0/2.0, more preferably 1.0/1.5 to 1.0/1.8.

7. The process according to any previous claim, wherein, in said step of pre-treating said waste textile material with said aqueous solution comprising NaOH and urea, the amount by weight of said waste textile material with respect to the volume of said aqueous solution comprising NaOH and urea is in the range from 1% w/v from 20% w/v, preferably from 2% w/v to 15% w/v, more preferably from 2.5% w/v to 10% w/v, most preferably 5% w/v.

8. The process according to any previous claim, wherein said step of pre-treating said waste textile material with said aqueous solution comprising NaOH and urea is carried out at a temperature within the range from -25°C to +30°C, preferably -15°C to +25°C, preferably from 4°C to +25°C, most preferably in a range of +15°C to +25°C.

9. The process according to any previous claim, wherein said pre-treating step is carried out for a time in the range from 0.5 hours to 10 hours, preferably from 1 hour to 8 hours, more preferably from 2 hours to 7 hours.

10. The process according to any previous claim, wherein said cellulase has an enzymatic activity from 1 FPU per gram of cellulose to 50 FPU per gram of cellulose, preferably from 10 FPU per gram of cellulose to 30 FPU per gram of cellulose, more preferably from 15 FPU per gram of cellulose to 25 FPU per gram of cellulose, and optionally wherein said cellulase is in the form of a solution comprising cellulase enzymes.

11. The process according to any previous claim, wherein said waste textile material includes a dye, at least part of said dye is removed from said waste textile material before said step c), preferably before said step b), or it is removed from said solid phase or from said liquid phase of said mixture including a solid phase and a liquid phase, after step d).

12. The process according to any previous claim, wherein said non-cellulosic fibers are recovered from said solid phase of said mixture including a solid phase and a liquid phase.

13. The process according to any previous claim, wherein said mixture including glucose as obtained from step d) is thermally treated to inactivate said cellulase enzymes before it is used in step e), whereby said inactivated enzymes provide a source of nutrients for said microorganisms.

14. The process according to claim 1 or any claim 3 to 13, wherein the glucose concentration in said mixture including glucose is in the range from 10 g/L to 30 g/L, preferably from 15 g/l to 25 g/L and the amount of said mixture including glucose is from 99% to 80% by weight, preferably from 95% to 85% by weight of the microbial culture weight.

15. The process according to claim 1 or any claim 3 to 14, wherein said mixture including glucose is mixed with a starter microbial culture comprising microbial cellulose-producing microorganisms, the amount of said starter microbial culture comprising microbial cellulose-producing microorganisms is from 1% to 20% by weight, preferably from 5% to 15% by weight of the microbial culture weight.

16. The process according to claim 1 or to any claim 3 to 15, wherein said step of preparing a microbial culture using said mixture including glucose comprises a step of adding at least one additive to said mixture including glucose, wherein said additive is preferably selected from titanium (IV) bis-(ammonium lactate) dihydroxide (TiBALDH), glycerol, ascorbic acid, ethephon (2-chloroethylphosphoric acid) and mixtures thereof.

17. The process according to claim 1 or to any claim 3 to 16, wherein said microbial cellulose is washed and, optionally, dried, wherein said washing is carried out using an aqueous solution including a bleaching agent preferably NaOCl in an amount in the range from 0.01% to 10% (w/v), preferably from 0.1% to 0.05% (w/v).

18. The process according to claim 1 or to any claim 3 to 17, wherein said microbial cellulose is used to produce cellulosic fibers.

19. A process of recycling blend textiles that include polyester fibers or filaments and cellulosic fibers, comprising the following steps:

   g) providing a waste textile material;
   h) pre-treating said waste textile material with an aqueous solution comprising NaOH and urea, to obtain a pre-treated textile material, wherein said step of pre-treating said waste textile material with an aqueous solution comprising NaOH and urea is carried out at a temperature in the range from -25°C to +30°C;

i) treating said pre-treated textile material with cellulase, to convert said cellulose into glucose, whereby a mixture including a solid phase including polyester fibers or filaments and a liquid phase is obtained;

j) separating said liquid phase from said solid phase, to obtain a liquid mixture including glucose,

k) recovering said polyester fibers or filaments from said solid phase.

20. A process according to claim 19, further characterized according to any claim 3 to 12.

Fig. 1

before pre-treatment

after pre-treatment

Fig. 2

Fig. 3A

Fig. 3B

Fig. 4A

Fig. 4B

Fig. 5A

Fig. 5B

Fig. 5C

Europäisches
Patentamt

European
Patent Office

Office européen
des brevets

# EUROPEAN SEARCH REPORT

Application Number

EP 24 16 9761

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FENG HONG ET AL: "Bacterial cellulose production from cotton-based waste textiles: Enzymatic saccharification enhanced by ionic liquid pretreatment", BIORESOURCE TECHNOLOGY, ELSEVIER, AMSTERDAM, NL, vol. 104, 7 November 2011 (2011-11-07), pages 503-508, XP028351226, ISSN: 0960-8524, DOI: 10.1016/J.BIORTECH.2011.11.028 [retrieved on 2011-11-25] * page 504 - page 505 * ----- | 1-20 | INV. C12N1/20 C12P19/04 C08B16/00 C08J11/10 |
| X | CHIA-HUNG KUO ET AL: "Enzymatic saccharification of dissolution pretreated waste cellulosic fabrics for bacterial cellulose production by Gluconacetobacter xylinus", JOURNAL OF CHEMICAL TECHNOLOGY AND BIOTECHNOLOGY, vol. 85, no. 10, 9 September 2010 (2010-09-09), pages 1346-1352, XP055300976, Hoboken, USA ISSN: 0268-2575, DOI: 10.1002/jctb.2439 * page 1347 - page 1351 * ----- | 1-12 | TECHNICAL FIELDS SEARCHED (IPC) C12N C12P C08B C08J C09J |
| A | CN 111 269 953 A (UNIV WUHAN TEXTILE) 12 June 2020 (2020-06-12) * the whole document * ----- -/-- | 1-20 | |

The present search report has been drawn up for all claims

| Place of search | Date of completion of the search | Examiner |
|---|---|---|
| The Hague | 6 August 2024 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 24 16 9761

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WANG ET AL: "Effect of enzymatic treatment on cotton fiber dissolution in NaOH/urea solution at cold temperature", CARBOHYDRATE POLYMERS, APPLIED SCIENCE PUBLISHERS , LTD BARKING, GB, vol. 72, no. 1, 11 January 2008 (2008-01-11), pages 178-184, XP022418407, ISSN: 0144-8617, DOI: 10.1016/J.CARBPOL.2007.08.003 * page 179 - page 180 * | 1 | |

TECHNICAL FIELDS
SEARCHED     (IPC)

The present search report has been drawn up for all claims

| Place of search | Date of completion of the search | Examiner |
|---|---|---|
| The Hague | 6 August 2024 | Oderwald, Harald |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
......................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 16 9761

This annex lists the patent family members relating to the patent documents cited in the above-mentioned European search report.
The members are as contained in the European Patent Office EDP file on
The European Patent Office is in no way liable for these particulars which are merely given for the purpose of information.

06-08-2024

| Patent document cited in search report | Publication date | Patent family member(s) | Publication date |
|---|---|---|---|
| CN 111269953 A | 12-06-2020 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102080114

- CN 111269953

**Non-patent literature cited in the description**

- **CHIA-HUNG KUO et al.** Enzymatic saccharification of dissolution pretreated waste cellulosic fabrics for bacterial cellulose production by Gluconacetobacter xylinus. *JOURNAL OF CHEMICAL TECHNOLOGY AND BIOTECHNOLOGY,* vol. 85 (10), 1346-1352
- **ADNEY B. ; BAKER J.** Measurement of cellulase activity. National Renewable Energy Laboratory, 1996
- **SELIG M. ; WEISS N. ; JI Y.** Enzymatic saccharification of lignocellulosic biomass. National Renewable Energy Laboratory, 2008

- **GHOSE, T.K.** Measurement of Cellulase Activities. *Pure & Applied Chemistry,* 1987, vol. 59, 257-268
- **MILLER G.L.** Use of Dinitrosalicylic Acid Reagent for Determination of Reducing Sugar. *Analytical Chemistry,* 1959, vol. 31, 426-428
- **JASME, NURSHAFQAH et al.** First report of biocellulose production by an indigenous yeast, Pichia kudriavzevii USM-YBP2. *Green Processing and Synthesis.,* 2022, vol. 11 (1
- *CHEMICAL ABSTRACTS,* 65104-06-5
- *CHEMICAL ABSTRACTS,* 56-81-5
- *CHEMICAL ABSTRACTS,* 50-81-7